(19) Europäisches Patentamt
European Patent Office
Office européen des brevets

(11) **EP 4 721 861 A1**

(12) **EUROPEAN PATENT APPLICATION**

(43) Date of publication:
08.04.2026 Bulletin 2026/15

(21) Application number: 25206039.7

(22) Date of filing: 01.10.2025

(51) International Patent Classification (IPC):
**B01L 3/00** (2006.01)  **C12Q 1/6806** (2018.01)

(52) Cooperative Patent Classification (CPC):
**B01L 3/502761; C12Q 1/6806;** B01L 2200/0663;
B01L 2200/0668; B01L 2300/0645;
B01L 2400/0415; B01L 2400/086

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 01.10.2024 US 202463702095 P
26.11.2024 US 202418960769

(71) Applicants:
• **Robert Bosch GmbH**
**70442 Stuttgart (DE)**
• **The Board of Trustees of the Leland Stanford
Junior University
Stanford, CA 94305-2038 (US)**

(72) Inventors:
• **SANTIAGO, JUAN G.
Stanford, CA, 94305-2038 (US)**
• **MA, KUNLIN
Stanford, CA, 94305-2038 (US)**
• **PAUL, SOUMYADEEP
Stanford, CA, 94305-2038 (US)**
• **DARVISH, ARMIN
Sunnyvale, CA, 94085 (US)**
• **LANG, CHRISTOPH
Sunnyvale, CA, 94085 (US)**

(74) Representative: **Isarpatent
Patent- und Rechtsanwälte
Barth Hassa Peckmann & Partner mbB
Friedrichstraße 31
80801 München (DE)**

(54) **METHODS FOR TRAPPING POLYNUCLEOTIDE STRANDS AT A DEFINED SURFACE**

(57) Systems and methods for trapping a polynucleotide strand at a defined surface of a channel. Methods may include providing a device including a channel and providing to a defined surface of the channel a polymer capable of modifying an electroosmotic flow in the channel and a sample including a polynucleotide strand. Methods may also include physically interacting the polymer with the defined surface of the channel and applying an electric field to the defined surface of the channel to promote a physical interaction between the polynucleotide strand and the polymer to trap the polynucleotide strand at the defined surface of the channel.

FIG. 6

**Description**

CROSS REFERENCE

This application claims the benefit of U.S. provisional application Serial No. 63/702,095 filed October 1, 2024, the disclosures of which are hereby incorporated in their entirety by reference herein.

TECHNICAL FIELD

The present disclosure relates to systems and methods of controlling, trapping, and stretching polynucleotide strands at a defined surface.

BACKGROUND

Single-molecule DNA flow and migration control, trapping, stretching, and imaging may enable fundamental studies of DNA polymer physics and DNA interaction with flow and electric fields. Current techniques require complex fabrication, lack throughput and are not cost-effective. Additionally, current methods analyze moving molecules, often within nanometer scale channels, which reduces detection quality. As such, there is a need for systems and methods of promoting control, capture, and visualization of single-molecule polynucleotide strands that are cost effective and allow for high-throughput processing of samples.

SUMMARY

In at least an aspect, a method of trapping a polynucleotide strand at a defined surface of a channel is provided. The method may include providing a device including a channel; providing to a defined surface of the channel a polymer capable of modifying an electroosmotic flow in the channel and a sample including a polynucleotide strand; physically interacting the polymer with the defined surface of the channel; and applying an electric field to the defined surface of the channel to promote a physical interaction between the polynucleotide strand and the polymer to trap the polynucleotide strand at the defined surface of the channel. The channel may be a microfluidic channel. The method may further comprise using flow focusing to direct the polymer to the defined surface of the channel. The method may further comprise shaping the channel walls and/or adding features to the channel walls to modify the magnitude of the electric field at the defined surface. The defined surface may be a dielectric material. The dielectric material may be an oxide. The channel surface outside of the defined surface may be a non-oxide material. The non-oxide material may be a metal. The polymer may be capable of adsorbing to the oxide material and may not be capable of adsorbing to the non-oxide material. The polymer may be a neutral water-soluble polymer. The polymer may be a polyvinylpyrrolidone polymer of greater than 100 kDa. The polymer may be a neutral water-soluble polymer. The polymer may be a polyvinylpyrrolidone polymer of greater than 100 kDa.

BRIEF DESCRIPTION OF THE DRAWINGS

Figures 1A and 1B illustrate methods for trapping and stretching a polynucleotide strand.

Figures 2A and 2B illustrate methods for trapping and stretching a polynucleotide strand.

Figures 3A and 3B illustrate systems for trapping and stretching a polynucleotide strand.

Figure 4 illustrates a channel with more than one fluid layer.

Figure 5 illustrates an example of a channel having an area with a low electric (E) and an area with a high electric field.

Figure 6 illustrates an example of a channel having a surface to which a polymer may adsorb, and defined areas of patterned material to which the polymer does not adsorb.

Figures 7A and 7B illustrate a method of optical imaging of a vertex trapped polynucleotide strand for the purpose of determining a sequence of the polynucleotide strand.

Figure 8 illustrates a system for optical imaging of a vertex trapped polynucleotide strand for the purpose of determining a sequence of the polynucleotide strand.

Figures 9A through 9C illustrate the method of DNA trapping according to an embodiment.

Figures 10A through 10B illustrate a flow chart summary of image processing used to quantify an amount of DNA trapping.

Figures 11A and 11B illustrate an analysis of relaxation dynamics of vertex-pinned DNA upon removal of the electric field. Figures 11C and 11D show statistical moments of characteristic lengths of pinned DNA molecules as they relax.

Figure 12 shows a representative cycle of DNA vertex-pinning at high electric field.

Figures 13A through 13G show that single DNA molecules are vertex-pinned to walls at locations which vary randomly from realization to realization.

Figures 14A through 14F illustrate a custom microfluidic interface device according to an embodiment.

Figures 15A and 15B illustrate DNA vertex-pinning at bottom and top surface of a 3 $\mu m$ deep straight channel under axial electric field strength of 150 V/cm.

Figure 16A illustrates a schematic of the commercial glass chip purchased from Microfluidic ChipShop. Figures 16B and 16C illustrate consecutive epifluorescence raw images of DNA electromigrating through a 37 $\mu m$ deep commercial glass channel. Figure 16D illustrates a global temporal median of the image sequence.

Figure 17 illustrates vertex-pinned, single-molecule 20 kbp DNA in a 0.9 $\mu m$ deep microfluidic channel filled with a linear polymer buffer solution and subject to an applied axial electric field.

Figures 18A through 18D illustrate a comparison between area-averaged alpha-shape-bounded intensity (*I*) versus results from manual counting of molecules.

Figure 19 illustrates an experimental quantification of the amount of vertex-pinned, single-molecule DNA as a function of field strength.

Figures 20A through 20D illustrate an experimental quantification of the amount of vertex-pinned, single-molecule DNA as a function of time and field strength.

Figure 21 illustrates the relaxation dynamics of vertex-pinned, single-molecule DNA.

Figures 22A through 22J illustrate a summary of automated image processing used to quantify 48.5 kbp DNA relaxation.

Figures 23A through 23J illustrate a summary of automated image processing used to quantify 20 kbp DNA relaxation.

Figures 24A through 24C illustrate a colocalization analysis of image intensity of vertex-pinned DNA across three different realizations.

Figures 25A through 25C illustrate a colocalization analysis of image intensity of vertex-pinned DNA across three consecutive images from one realization.

Figure 26 illustrates example frames from this video that show the difference between raw video and the moving median video, which is processed to only show non-moving DNA molecules.

## DETAILED DESCRIPTION

As required, detailed embodiments of the present disclosure are disclosed herein; however, it is to be understood that the disclosed embodiments are merely exemplary of the disclosure that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present disclosure.

Except in the examples, or where otherwise expressly indicated, all numerical quantities in this description indicating amounts of material or conditions of reaction and/or use are to be understood as modified by the word "about". The first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

Unless indicated otherwise, all technical and scientific terms used herein have the same meaning as commonly understood by one of ordinary skill in the art to which the present disclosure belongs.

It is also to be understood that this disclosure is not limited to the specific embodiments and methods described below, as specific components and/or conditions may, of course, vary. Furthermore, the terminology used herein is used only for describing particular embodiments and is not intended to be limiting in any way.

It must also be noted that, as used in the specification and the appended claims, the singular form "a," "an," and "the" comprise plural referents unless the context clearly indicates otherwise. For example, reference to a component in the singular is intended to comprise a plurality of components.

The terms "or" and "and" can be used interchangeably and can be understood to mean "and/or".

The term "comprising" is synonymous with "including," "having," "containing," or "characterized by." These terms are inclusive and open-ended and do not exclude additional, unrecited elements or method steps.

The phrase "consisting of" excludes any element, step, or ingredient not specified in the claim. When this phrase appears in a clause of the body of a claim, rather than immediately following the preamble, it limits only the element set forth in that clause; other elements are not excluded from the claim as a whole.

The phrase "consisting essentially of" limits the scope of a claim to the specified materials or steps, plus those that do not materially affect the basic and novel characteristic(s) of the claimed subject matter.

The terms "polynucleotide", "nucleotide", "nucleotide sequence", "nucleic acid", "polynucleic acid", and "oligonucleotide" may be used interchangeably in this disclosure. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: single-, double-, or multi-stranded DNA or RNA, genomic DNA, cDNA, DNA amplicon products, DNA restriction products, synthetic non-natural polynucleotide strands including peptide nucleic acids (PNA) and locked nucleic acids (LNA), DNA-RNA hybrids, or a polymer comprising purine and pyrimidine bases or other natural, chemically or biochemically modified, non-natural, or derivatized nucleotide bases. The terms "polynucleotide" and "nucleic acid" should be understood to include, as applicable to the embodiment being described, singlestranded (such as sense or antisense) and double-stranded polynucleotides. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

The terms "complementarity" or "complement" refers to the ability of a nucleic acid to form hydrogen bond(s) with another nucleic acid sequence by either traditional Watson-Crick or other non-traditional types. A percent complementarity indicates the percentage of residues in a nucleic acid molecule which can form hydrogen bonds (e.g., Watson-Crick base pairing) with a second nucleic acid sequence (e.g., 4, 5, and 6 out of 6 being 66.67%, 83.33%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 40%, 50%, 60%, 62.5%, 70%, 75%, 80%, 85%, 90%, 95%, 97%, 98%, 99%, or 100%, or percentages in between over a region of 4, 5, 6, 7, and 8 nucleotides, or refers to two nucleic acids that hybridize under stringent conditions.

Unless expressly stated to the contrary: the term "polymer" includes "oligomer," "copolymer," "terpolymer," and the like; molecular weights provided for any polymers refers to weight average molecular weight unless otherwise indicated; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

It should also be appreciated that integer ranges explicitly include all intervening integers. For example, the integer range 1-10 explicitly includes 1, 2, 3, 4, 5, 6, 7, 8, 9, and 10. Similarly, the range 1 to 100 includes 1, 2, 3, 4. . . . 97, 98, 99, 100. Similarly, when any range is called for, intervening numbers that are increments of the difference between the upper limit and the lower limit divided by 10 can be taken as alternative upper or lower limits. For example, if the range is 1.1. to 2.1 the following numbers 1.2, 1.3, 1.4, 1.5, 1.6, 1.7, 1.8, 1.9, and 2.0 can be selected as lower or upper limits. In the specific examples set forth herein, concentrations, temperature, and reaction conditions (e.g. pressure, pH, etc.) can be practiced with plus or minus 50 percent of the values indicated rounded to three significant figures. In a refinement, concentrations, temperature, and reaction conditions (e.g., pressure, pH, etc.) can be practiced with plus or minus 30 percent of the values indicated rounded to three significant figures of the value provided in the examples. In another refinement, concentrations, temperature, and reaction conditions (e.g., pH, etc.) can be practiced with plus or minus 10 percent of the values indicated rounded to three significant figures of the value provided in the examples.

In the examples set forth herein, concentrations, temperature, and reaction conditions (e.g., pressure, pH, flow rates, etc.) can be practiced with plus or minus 50 percent of the values indicated rounded to or truncated to two significant figures of the value provided in the examples. In a refinement, concentrations, temperature, and reaction conditions (e.g., pressure, pH, flow rates, etc.) can be practiced with plus or minus 30 percent of the values indicated rounded to or truncated to two significant figures of the value provided in the examples. In another refinement, concentrations, temperature, and reaction conditions (e.g., pressure, pH, flow rates, etc.) can be practiced with plus or minus 10 percent of the values indicated rounded to or truncated to two significant figures of the value provided in the examples.

Single-molecule DNA trapping and stretching may enable fundamental studies of DNA polymer physics and DNA interaction with flow and electric fields. For such studies, methods to trap and stretch single-molecule DNA with simultaneous visualization have included using optical or magnetic tweezers to manipulate microbeads tethered to DNA; subjecting DNA to extensional flow strain in microfluidic devices; incorporating linking chemistries to tether DNA to flat surfaces and then subjecting them to fluid flow or electric fields, as in so-called DNA curtains; and so-called combing of DNA on silanized glass using a moving air/water interface. Confining and trapping of single-molecule DNA combined with high-quality imaging has also been applied to genetic analyses, including karyotyping and DNA mapping. For example, electromigration within nanochannels and imaging of long (>100 kb) genomic DNA with sequence-specific labels is used for optical mapping in medical diagnostics.

Such techniques have certain disadvantages, however. For instance, they lack throughput. Optical and Magnetic Tweezers can study a single molecule or at best a small number of molecules. These techniques additionally require expensive complex imaging and control systems. Molecular combing and DNA curtain assays can image many molecules but cannot cycle through multiple samples. These assays therefore cannot resample the same population or assay new samples. In this way, multiplexing the data analysis is necessary to capture the information from a fast-moving molecule. Additionally, because the molecules in these techniques are non-stationary (moving molecules), it is difficult to obtain high quality images or to study dynamic interactions. Other difficulties of using electromigration within nanochannels (including so-called nanoslit channels) for DNA isolation include difficulty in fabricating and sealing channels and difficulty in controlling flow in these systems including filling with liquid, creating pressure-driven flow, and creating electrokinetic flows. Also, fabrication methods for nanochannels may result in poor optical access into the channels. Further, the walls of such nanochannels may have roughness elements which are on the order of the cross-sectional dimensions of the channels. Another challenge is that nanochannels can also pose non-uniformities in surface characteristics which can lead to non-uniform zeta potentials and therefore non-uniform electroosmotic flow velocities which impart velocity gradients in the fluid. Current methods for trapping DNA (with and without visualization of DNA) including optical and magnetic tweezers as described above and/or adsorbing DNA onto surfaces using liquid/air interfaces using so-called DNA combing or DNA curtain techniques may require tedious manual steps and are not compatible with high throughput applications where many DNA molecules need to be individually trapped and stretched. As such, there is a need for systems and methods of promoting control and detection of single-molecule polynucleotide strands that are cost effective and allow for high-throughput processing of samples.

Systems and methods for trapping, stretching, release, and/or re-trapping of many single-molecule polynucleotide strands are disclosed herein. The disclosed systems and methods may promote control and detection (including visualization) of single-molecule polynucleotide strands.

Methods of trapping, stretching, release, and/or re-trapping of many single-molecule polynucleotide strands are disclosed herein. Polynucleotide strands may refer to a strand of linked nucleotides and may include DNA or RNA for example. A polynucleotide strand may be referred to as a strand or as a molecule. One or more of the methods may promote single-molecule control and visualization. According to various method embodiments, a polynucleotide strand in a sample may be pinned at a vertex along its length to a wall of a microchannel. The polynucleotide strand may be trapped, held in place, and stretched using an applied axial electric field. This may enable high quality imaging and quantification of polynucleotide strand conformation. In one or more embodiments, this disclosure presents a set of conditions for a polynucleotide strand to be trapped; quantifies trapping dynamics; and quantifies relaxation dynamics, disentanglement, and release from the pinned state.

According to at least an embodiment, a method for trapping and stretching a polynucleotide strand is disclosed. The method promotes trapping a polynucleotide strand at a surface of a channel. The channel may be a microfluidic channel. The term channel, as used here, also includes fluidic chambers which are in fluid communication with at least one port which may be used to fill the chamber with liquid. The channel may be part of a microfluidic device including one or more channels. The one or more channels may each have a single input and a single output. There may be multiple independent channels. Samples in each of the independent channels may be processed in parallel. The surface of the channel may be dielectric materials including oxides. For example, the channel surface may be silicon oxide, aluminum oxide, titanium oxide, or various other oxide surfaces. The channel surface may also be a nitride such as silicon nitride for example. The surface may also be glass including borosilicate glass, or quartz. The channel surface may also be dielectric with areas of non-dielectric material (e.g. metals or semiconductors). The dielectric and non-dielectric materials may be arranged in a particular pattern on the surface. The polynucleotide strands may be trapped at the dielectric areas but not at the non-dielectric areas. The channel may also include a channel cover. The channel cover may be borosilicate glass. The channel may include a floor and one or more walls extending outward from the floor. The walls and/or cover of the channel may be flat. The surface of the channel may include surfaces etched into a substrate or the channel cover. The surface of the channel may alternatively include a defined portion of the substrate or of any of the walls of the channel, or of the channel cover. In certain embodiments, the channel may have a depth from 500 nm to 3 $\mu$m. A channel depth in this range may for example promote easy acquisition of high quality images of the polynucleotide strands via epifluorescence microscopy. In other embodiments, the channel may have a depth greater than 3 $\mu$m. High quality images of the polynucleotide strands may be acquired via (Total Internal Reflection Fluorescence) TIRF microscopy with a channel depth greater than 3 $\mu$m for example. In various embodiments, the channel may also have a nominal spanwise width larger than 1 $\mu$m. The channel may optionally include one or more posts to support the cover and/or a castellation pattern on one or more side walls of the channel. The channel may have at least one dimension normal to the applied electric field of less than 5 microns.

The transport of reagents and/or liquid in the system may be accomplished using processes including electromigration, electroosmotic flow, pressure-driven flow, dielectrophoresis, magnetophoresis, electrowetting, induced charge electroosmotic flows, alternating current (AC) electrokinetic flows, gravity-driven flows, and surface-tension driven flows.

The method may include providing a polymer to the surface of the channel. The polymer is capable of suppressing an electroosmotic flow in the channel. The polymer may be a neutral water-soluble linear polymer. The polymer may be polyvinylpyrrolidone (PVP) for example. PVP is a neutral water-soluble linear polymer that may be used as an additive in a buffer system to suppress electroosmotic flow (EOF) to perform electrokinetic experiments. The polymer may alternatively be polyethylene glycol (PEG), which may also be known as polyethylene oxide (PEO) in the context of polymer chemistry for example. PEG may be used in gas chromatography and may also be used to coat or treat a silica surface to suppress EOF. PEG with large enough molecular weight may also enable vertex-pinning of DNA. The polymer may alternatively be hydroxyethyl cellulose (HEC). HEC may suppress EOF as a linear polymer. The polymer may alternatively be polyvinyl alcohol (PVA). PVA may be used for separation of DNA restriction fragments and effectively suppresses EOF below about pH 8.

The method may additionally include physically interacting the polymer with a surface of the channel. This step may be achieved by coating the surface with the polymer. Coating may be achieved by delivering the polymer to the surface and allowing the polymer to adsorb to the surface. The surface may include a floor or a wall of the channel, a portion of the floor or a wall of the channel, the channel cover or a portion of the channel cover, or a surface not in physical contact with the channel cover. For example, the polymer may be capable of adsorbing to the silicon oxide walls of the channel or to the borosilicate glass walls of the channel cover. In at least some embodiments, metal to which the polymer does not adsorb may be deposited on the oxide substrate in a pattern. In this way, only the areas of the oxide substrate (e.g., not patterned with metal) with sufficient electric field and the presence of a linear polymer interacting with the surface may trap polynucleotide strands.

The method may further include providing a sample including a polynucleotide strand to the polymer at a surface of the channel. The sample may include a plurality of polynucleotide strands. The polynucleotide strands may be isolated or synthetic. The polynucleotide strands may originate from the same or different sources. For example, different sources may include polynucleotide strands from different organism (e.g., a human) or from the same organism. A single sample may include polynucleotide strands from both control and experimental sources. The polynucleotide strands in the sample may include a barcode that identifies the source or type of the polynucleotide strand. Sources may include, for example, human, plant, or animal cells or tissue, microphysical systems including organoids, spheroids, tumoroids and similar systems, bacterial or protozoan cells, or a synthetic reaction that produces polynucleotide strands. The sample including the polynucleotide strand may additionally include one or more buffer components. In at least one embodiment, the sample provided to the channel may include a polynucleotide strand, buffer components, and the polymer.

The method additionally includes applying an electric field to the polynucleotide strand to promote a physical interaction between the polynucleotide strand and the polymer to trap (also referred to as pinning in one or more embodiments) the polynucleotide strand at the surface of the channel. The electric field may be from 30 to 400 V/cm. The electric field may be applied to an electrolyte by two or more electrodes which support Faraday reactions. These Faraday reactions can support ionic currents in the channel. The applied field associated with these ionic currents can be substantially parallel to walls on which the polynucleotide strand is to be trapped. For example, the interior wall of the channel can be a dielectric material such that the applied electric field is parallel to the wetted wall of the channel and drives electromigration (including electrophoresis) of polynucleotide strands parallel to the wall of the channel. Alternately, the electric field which promotes the interaction between the polynucleotide strand and the wall may be applied using electrodes which are capacitively coupled to the electrolyte and thereby avoid or mitigate Faraday reactions in the system. The electric field may be applied to the channel, thereby affecting the channel as well as the contents of the channel including any fluid samples. For example, applying the electric field to the channel may result in the application of the electric field to the polynucleotide strands in the sample flowing through the channel. The physical interaction between the polynucleotide strand and the polymer may occur at a vertex of the polynucleotide strand. The physical interaction may occur at a single vertex along the length of the polynucleotide strand. The two ends (also referred to as arms) of the polynucleotide may stretch outwardly from the vertex in the opposite direction of the applied electric field.

The trapping of a polynucleotide strand via vertex pinning and stretching occurs when a polymer having a sufficiently large molecular weight (greater than 100 kDa for example) is provided to the surface of the channel, and when a sufficiently large axial electric field (>30 V/cm for example) is applied. The threshold for the electric field may be smaller for a polymer of larger molecular weight. For example, the threshold for the electric field is lower for larger molecular weight PVP polymer. The trapping and stretching phenomena occur only at the surface and occur on both thermal silicon oxide and glass surfaces.

Without favoring any particular theory, at sufficiently high electric fields, polynucleotide strands may become entangled with surface-adsorbed polymer, and the strong electric forces may expel a hydration layer between the nucleic acid and the polymer, resulting in a high friction condition. Upon removal of the electric field, the space between the nucleic acid and the polymer may become re-hydrated, and the resulting lower friction may enable Brownian motion to disentangle the polynucleotide strand from the polymer. No spatial correlation of vertex pin-points occurs across realizations, suggesting that the phenomenon is not associated with specific channel geometry.

The method may also include lowering the electric field below an empirically determined threshold or removing the electric field to promote release of the polynucleotide strand from the physical interaction with the polymer. Upon removal of an electric field above an empirically determined threshold, a polynucleotide strand may relax toward and around the vertex of the pinned location. The pinned location may be a single-point entanglement between the polynucleotide strand and the polymer. After the polynucleotide strand relaxes toward and around the vertex of the pinned location, it may disentangle from the polymer and resume moving through the channel. The movement of the disentangled polynucleotide strand through the channel may occur through diffusion or electromigration. The relaxing, disentangling and movement of the polynucleotide strand may clear the polynucleotide strand from the channel so that a second sample including a polynucleotide strand may be provided to the channel for trapping and stretching. In this way, the method may include a cyclical arrangement of steps including: introduction of a polynucleotide strand into a region of interest, trapping a polynucleotide strand at high electric field, imaging the polynucleotide strand one or more times (including detecting various wavelengths, lowering the electric field to relax the polynucleotide strand which may lead to disentanglement, applying an electric field which causes the polynucleotide strand to electromigrate with very little or no capture, and then repeating the process. In this way, a next sample including a polynucleotide strand may be input into the channel. For example, after a first sample has been trapped, relaxed, disentangled, and cleared, a second sample may be provided to the channel for trapping. After that sample has been trapped, relaxed, disentangled, and cleared a next sample may be provided to the channel for trapping. The process of trapping, relaxing, disentangling, and clearing may be repeated for n samples. This cyclical arrangement may allow for high-throughput processing of many independent samples and may also allow for resampling of the same sample multiple times to enhance the output. The second sample, next sample, or nth sample may include polynucleotide strands that were present in the first or previous sample. Further, a pulse of pressure-driven flow may be introduced which would cause much faster disentanglement. In some embodiments, the step of deactivating the electric field may be replaced with a step of providing a pulse of pressure driven flow to the channel. The cyclical arrangement of steps in the method may enable high-throughput trapping and analysis of many polynucleotide strands.

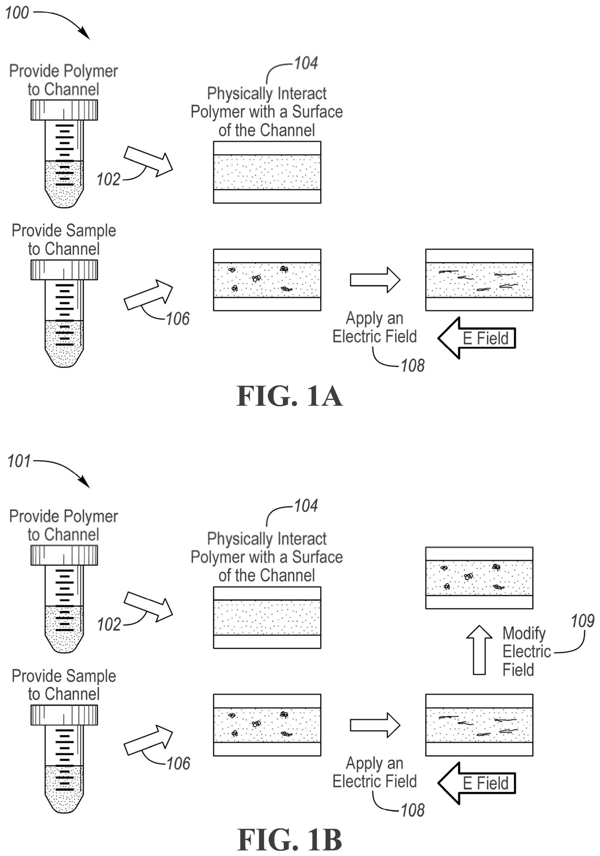

Figure 1A illustrates a method 100 for trapping and stretching a polynucleotide strand according to an embodiment. The method includes a step 102 of providing a polymer to the surface of a channel. The method includes an additional step 104 of physically interacting the polymer with the surface of the channel. A step 106 includes providing a sample including the polynucleotide strand to the polymer interacting with the surface of the channel. The method additionally includes a step 108 of applying an electric field to the polynucleotide strand to promote a physical interaction between the polynucleotide strand and the polymer to trap the polynucleotide strand at the surface of the channel.

Figure 1B illustrates a method 101 for high throughput trapping and stretching a polynucleotide strand according to an embodiment. The method may promote trapping, stretching, and releasing a polynucleotide so that multiple samples may be sequentially processed. The method includes a step 102 of providing a polymer to the surface of a channel. The method includes an additional step 104 of physically interacting the polymer with the surface of the channel. A step 106 includes providing a sample including the polynucleotide strand to the polymer interacting with the surface of the channel.

The method additionally includes a step 108 of applying an electric field to the polynucleotide strand to promote a physical interaction between the polynucleotide strand and the polymer to trap the polynucleotide strand at the surface of the channel. Step 109 includes modifying the electric field to release the trapped polynucleotide strand from the physical interaction with the polymer. Lowering the electric field may lead to relaxing of the polynucleotide strand which in turn leads to disentanglement and free diffusion and electromigration to clear the polynucleotide strand so that a next sample including a polynucleotide strand may be input into the channel. The steps of the method may then be repeated n times to achieve high throughput processing of polynucleotide strands. In an alternative embodiment, step 109 may include providing a pulse of pressure driven flow to the channel. The pulse may also release the polynucleotide strand by promoting relaxation, disentanglement, free diffusion and electromigration of the polynucleotide strand.

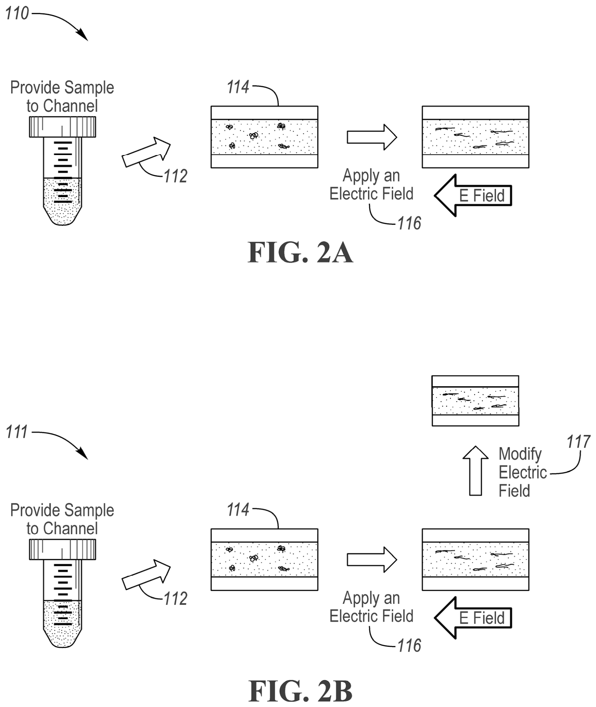

Figure 2A illustrates an additional method 110 for trapping and stretching a polynucleotide strand according to an embodiment. The method includes a step 112 of providing a sample including a polymer and a polynucleotide strand to a surface of a channel. A step 114 includes physically interacting the polymer with the surface of the channel. An additional step 116 includes applying an electric field to the polynucleotide strand to promote a physical interaction between the polynucleotide strand and the polymer to trap the polynucleotide strand at the surface of the channel.

Figure 2B illustrates an additional method 111 for high throughput trapping and stretching a polynucleotide strand according to an embodiment. The method may promote trapping, stretching, and releasing a polynucleotide so that multiple samples may be sequentially processed. The method includes a step 112 of providing a sample including a polymer and a polynucleotide strand to a surface of a channel. A step 114 includes physically interacting the polymer with the surface of the channel. An additional step 116 includes applying an electric field to the polynucleotide strand to promote a physical interaction between the polynucleotide strand and the polymer to trap the polynucleotide strand at the surface of the channel. Step 117 includes modifying the electric field to release the trapped polynucleotide strand from the physical interaction with the polymer. Lowering the electric field may lead to relaxing of the polynucleotide strand which in turn leads to disentanglement and free diffusion and electromigration to clear the polynucleotide strand so that the next sample including a polynucleotide strand may be input into the channel. The steps of the method may then be repeated n times to achieve high throughput processing of polynucleotide strands. In an alternative embodiment, step 117 may include providing a pulse of pressure driven flow to the channel. The pulse may also release the polynucleotide strand by promoting relaxation, disentanglement, free diffusion and electromigration of the polynucleotide strand.

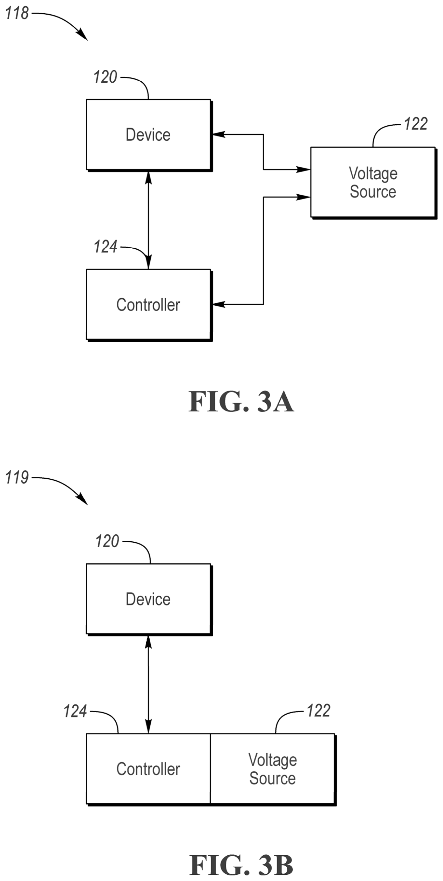

Figures 3A and 3B illustrate systems for trapping and stretching a polynucleotide strand. Figure 3A illustrates a system 118 including a device 120, a voltage source 122, and a controller 124 programmed to interact with the device 120 and the voltage source 122. The device 120 may include at least one reservoir sized to receive a sample including the polynucleotide strand. The reservoir may also include an electrode. Alternatively, the device 120 may include a first reservoir sized to receive a sample including the polynucleotide, and a second reservoir sized to receive an electrode. These reservoirs may be in electrical and/or ionic communication between themselves or among other electrodes in the system. The electrode (either alone in a reservoir, or in a reservoir along with the sample including the polynucleotide strand) may be metal, a metal alloy, or a semiconductor material for example. Other possible materials including graphite or graphene or other forms of carbon including activated porous carbon. Electrodes can be created using microfabrication techniques including evaporation, lift-off techniques, or doping. Electrodes can also be electroplated or coated. These electrodes may drive Faraday reactions (including water splitting at the electrode) to introduce electric fields within the channels of the system. The electrode may also have significant capacitance, including a porous carbon electrode operated at low voltage to avoid Faraday reactions. In at least some embodiments, the electrode may be a platinum electrode. One or more electrodes may be carbon-based, as mentioned earlier. For example, the electrode may be single or multi-layered graphene. The electrode may be graphite. The electrode may also be an inkjet-printed carbon deposited lead. Alternatively, the electrode may be gold, silver, titanium, palladium, copper, stainless steel, titanium nitride, or silver/silver chloride. Electrodes may also be fabricated from doped silicon. The voltage waveforms and resulting electric fields imposed by electrodes can be direct current (DC), alternating current (AC), pulsed-modulated, and can be very complex waveforms. For example, waveforms can include both DC and AC components and can be periodic, non-periodic, anti-periodic, or non-anti-periodic.

The device may also include a chip. The chip may be a silicon substrate chip. The chip may have two isolated (independent) single-input, single-output channels. The channels may be fabricated so that they can be visualized simultaneously within the same field of view near the geometric center of the silicon chip. The microfluidic channels may be dry-etched into the silicon wafer to a depth of 1 $\mu$m for example. The microfluidic channels may be in fluidic contact with the reservoir or reservoirs. There may be multiple independent channels. Samples in each of the independent channels may be processed in parallel. The device may also include a channel cover. The channel cover may be glass. The channel cover may for example be borosilicate glass. One or more walls of the channel may be optically clear and/or may promote efficient transmission to electromagnetic radiation such as infra-red or ultra-violet. One or more walls of the channel may also promote significant transmission, reflection, refraction, and/or diffraction of electromagnetic radiation. As defined herein, electromagnetic radiation includes optical (visible) wavelengths, infrared, and ultraviolet light. The system may permit and focus magnetic fields which may impose magnetic forces within the device, including magnetic particles.

The system may also include a voltage source, and a controller. The voltage source may apply the electric field to the polynucleotide strand in the channel. The voltage source may apply a voltage using the electrodes in the reservoirs. The voltage source may also measure current. The controller may be programmed to interact with the voltage source. For example, the controller may be programmed to direct the voltage source to apply a voltage or to measure current. The controller may be programmed to modify the electric field being applied to the channel. Additionally, the controller may be programmed to direct the flow of a polymer to the device to promote delivery of the polymer to the surface of the channel so that the polymer is capable of physically interacting with the surface of the channel. The controller may similarly direct the flow of the sample with or without the polymer to the device to promote delivery of the sample to the surface of the channel. The controller may also be programmed to direct an electromigration of the polynucleotide strand through the channel. Figure 3B illustrates a system 119 for trapping and stretching a polynucleotide strand according to an embodiment. The voltage source 122 is part of the controller 124.

Processes, methods, or algorithms disclosed herein can be deliverable to/implemented by a processing device, controller, or computer, which can include any existing programmable electronic control unit or dedicated electronic control unit. Similarly, the processes, methods, or algorithms can be stored as data and instructions executable by a controller or computer in many forms including, but not limited to, information permanently stored on non-writable storage media such as ROM devices and information alterably stored on writeable storage media such as floppy disks, magnetic tapes, CDs, RAM devices, and other magnetic and optical media. The processes, methods, or algorithms can also be implemented in an executable software object. Alternatively, the processes, methods, or algorithms can be embodied in whole or in part using suitable hardware components, such as Application Specific Integrated Circuits (ASICs), Field-Programmable Gate Arrays (FPGAs), state machines, controllers or other hardware components or devices, or a combination of hardware, software and firmware components.

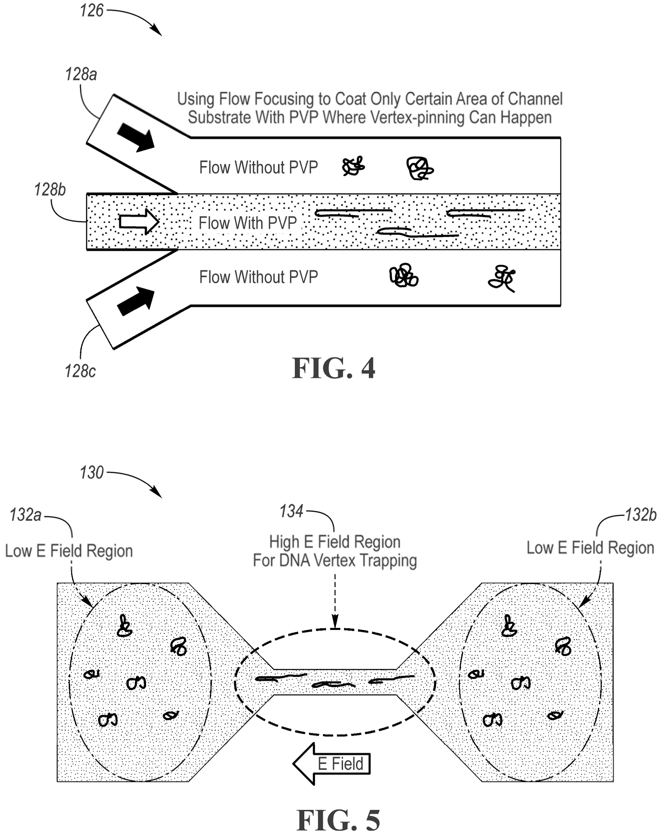

Methods for spatially patterning locations within a channel at which a polynucleotide strand may be trapped are also provided herein. Through these methods a polynucleotide may be trapped within a specific region of interest. In an embodiment, a method includes providing a polymer to specified locations within a fluidic system. For example, laminar flow features may be utilized to only provide the polymer to some sub-surfaces and to prevent the polymer from physically interacting with other surfaces. This method may utilize hydrodynamic focusing to pattern locations of wall surfaces treated by the polymer. Figure 4 illustrates a channel 126 with more than one fluid layer 128a, 128b, 128c. The first fluid layer 128a includes a polynucleotide strand but no polymer. The second fluid layer 128b includes both the polynucleotide strand and the polymer. The third fluid layer 128c includes the polynucleotide strand but no polymer. The polynucleotide is trapped in the second fluid layer 128b because the second fluid layer 128b includes the polymer. In the first layer 128a and the third layer 128c, the polynucleotide strand migrates in a 3D ball shape.

In alternative embodiments, a method for spatially patterning locations within a channel at which a polynucleotide strand may be trapped may include shaping the channel walls and/or adding features to affect the electric field in a specifically defined area. For example, obstructions may be added to locally affect the electric field in a channel. This method leverages the fact that polynucleotide strand trapping occurs above some threshold electric field. Figure 5 illustrates an example of a channel 130 having an area with a low electric (E) field 132a, 132b, and an area with a high electric field 134. The polynucleotide strands are only vertex pinned and stretched in the high E field region while polynucleotide strands in the low E field region migrate in 3D ball shapes.

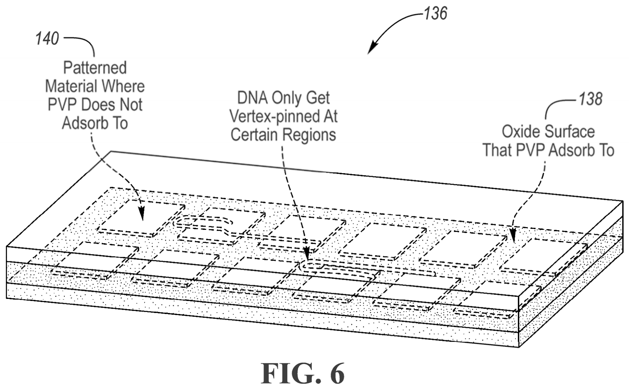

In yet other embodiments, a method for spatially patterning locations within a channel at which a polynucleotide strand may be trapped may include patterning metal coated areas on the fluidic chip where the polymers do not adsorb. Figure 6 illustrates an example of a channel 136 having a surface 138 to which a polymer may adsorb, and defined areas of patterned material 140 to which the polymer does not adsorb.

*Systems and methods for trapping, stretching, and detecting a polynucleotide strand*

The systems and methods disclosed in one or more embodiments herein describe systems and methods for vertex-pinning, relaxation, disentanglement and free electromigration cycling to trap and stretch single-molecule polynucleotide strands. Vertex pinning and stretching may be interspersed with relaxation, disentanglement, and low-electric-field clearing of the polynucleotide strands to cycle many polynucleotide strands. These systems and methods may additionally be modified to detect polynucleotide strands. For example, these systems and methods may be modified to achieve high-throughput capture, stretching, and imaging of polynucleotide strands. The systems and methods may also be modified for the purpose of determining a sequence of the polynucleotide strands. The systems and methods may promote trapping and stretching a polynucleotide strand at a specific region of interest within a channel. The specific region of interest may for example be a field of view within the channel.

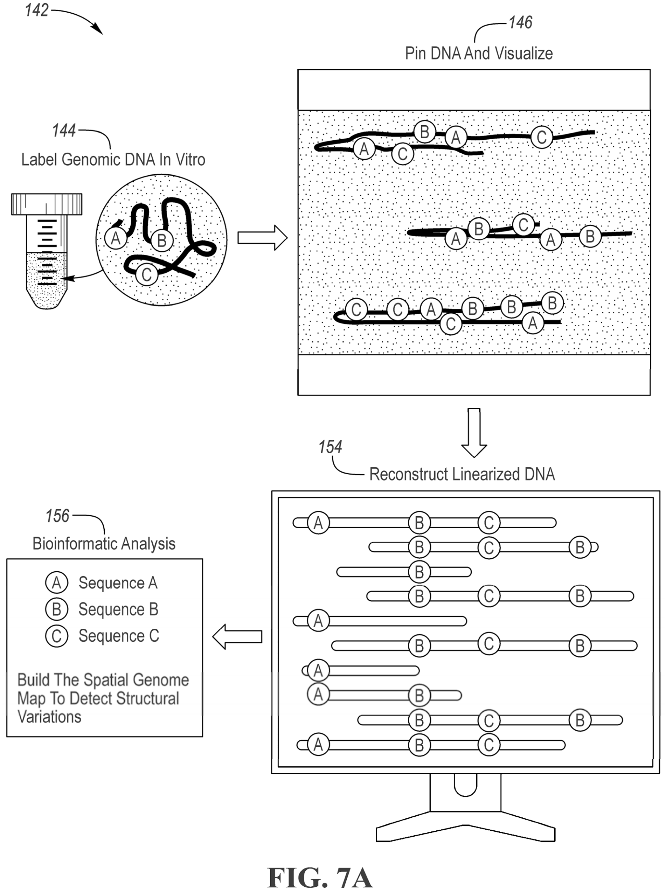

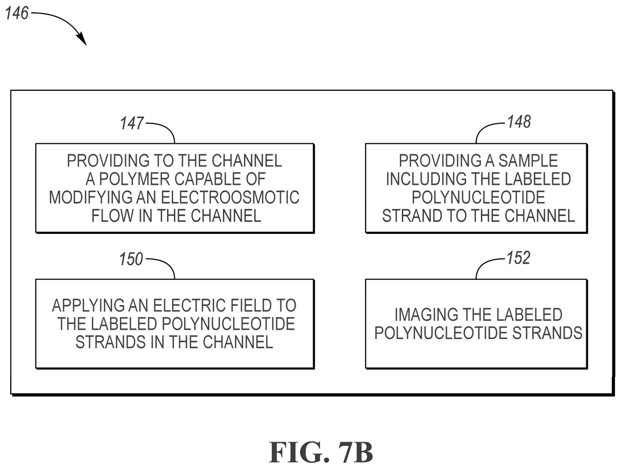

Figures 7A and 7B illustrate a method 142 of optical imaging of a vertex trapped polynucleotide strand for the purpose of visualizing a subsequence of the polynucleotide strand. A step 144 includes providing a sample including at least one polynucleotide strand and labeling the polynucleotide strand to create a labeled polynucleotide strand. The polynucleotide strand may be significantly greater than 1 kb. For example, the polynucleotide strand may be in excess of 300 kb in length. At least one sequence of four to 10 base pairs within the polynucleotide strand may be labeled to form a labeled subsequence. In this way, a labeled polynucleotide strand is a polynucleotide strand having at least one labeled subsequence. In an example, the polynucleotide strand may be DNA. The region containing the subsequence may be labeled with a fluorescent label. More than one subsequence may be labeled on the DNA strand. The subsequences may also be referred to as target sequences, subsequences, codes, or barcode elements. Labels of multiple types may be used including fluorescent labels with varying emission wavelengths. A step 146 includes trapping and visualizing the labeled polynucleotide strand. Step 146 includes the substeps 147, 148, 150, and 152. Substep 147 includes providing to the channel a polymer capable of modifying an electroosmotic flow in the channel. Substep 148 includes providing a sample including the labeled polynucleotide strand to the channel. In some embodiments, the polymer may be included in the sample including the labeled polynucleotide strand. The polymer may be a neutral and water-soluble polymer. An electric field above an empirically determined threshold is then applied to the polynucleotide strands in the channel (Substep 150). The threshold may depend on the polymer used and on the molecular weight of the polymer. The labeled polynucleotide strands then physically interact with the polymer at a surface of the channel and are trapped. Upon being trapped, the free ends (also referred to as arms) of the labeled polynucleotide strands stretch out in the direction opposite of the electric field. While trapped, the labeled polynucleotide strands may be imaged by a detector (Step 152). In an example, fluorescently labeled DNA strands may be imaged by an epifluorescence microscope and scientific complementary metal oxide semiconductor (scientic-CMOS) camera, charge-coupled device (CCD) or electron multiplying charge-coupled device (EMCCD) camera. Additional methods that may be used to image trapped polynucleotide strands (other than epifluorescence microscopy) include light scattering microscopy, total internal reflection microscopy (TIRF), super-resolution structural illumination microscopy (SR-SIM), stimulated emission depletion microscopy (STEP), stochastic optical reconstruction microscopy (STORM), or single-molecule localization microscopy (SMLM).

In at least an embodiment, labeled vertex-pinned DNA may be trapped and elongated under the applied electric field in a shallow microfluidic channel for high quality single molecule imaging. A step 154 includes programming a controller to collect, record, and store the signals obtained from the detector. The signals may then be processed (Step 156). In silico, the images of the DNA may be interpreted in order to analyze the sequence of labels. For example, the label-to-label distances form a pattern which can be detected and analyzed. Regions of trapped DNA which do not overlap with other DNA regions can be interpreted more easily than regions where one section of DNA overlaps with another section of DNA. Overlapped regions in the "hair pin" shape DNA can be analyzed in a computer (in silico) and the pattern of the DNA can be "unfolded" computationally so as to determine the pattern along the DNA in its unfolded form. That is, the pattern of labels associated with the unobserved unfolded DNA can be deduced from the imaged labels and may be computationally recovered.

As described above, applications may include imaging of fluorescently labeled markers on trapped DNA such as DNA mapping and detection of specific sequences. Such an application will require detection algorithms and methods designed to detect these codes. Given the nature of the vertex trapping and folded nature of DNA described here, there will be at least two classes of DNA shapes for DNA trapped in this manner. For many (and likely most) DNA entangled along some point along its length, there will be a region where the two DNA arms substantially overlap. In this overlap region, some sub-sequence oriented from 3' to 5' can largely overlap with some subsequence oriented from 5' to 3'. The two observable sub-sequences in this first overlap region converge near the vertex point. The two separate sequences in this overlapped region can be read by reading the overlapped region and then unfolding the contiguous sequence computationally. This would involve analysis (in a computer program) for overlaps of 3' to 5' which are nearby to 5' to 3' type sequences. Once the overlapped sequences are determined, the two overlapped regions can, in the computational domain, be interpreted.

In addition to this overlapped region, most DNA will also have a substantial second region further from the vertex where the longer DNA arm does not overlap with the shorter arm. This region contains a sub-sequence which can be either of the type 3' to 5' or of the 5' to 3' type. This sequence can be read then interpreted normally.

A method of interpreting overlapped DNA arm regions may include first identifying the non-overlapped region and then using this information to help interpret the overlapped region. An alternative method of interpreting overlapped DNA arm regions may include maintaining a database (in a computer) of codes associated with overlapped or partially overlapped regions and comparing the overlapped regions to these codes.

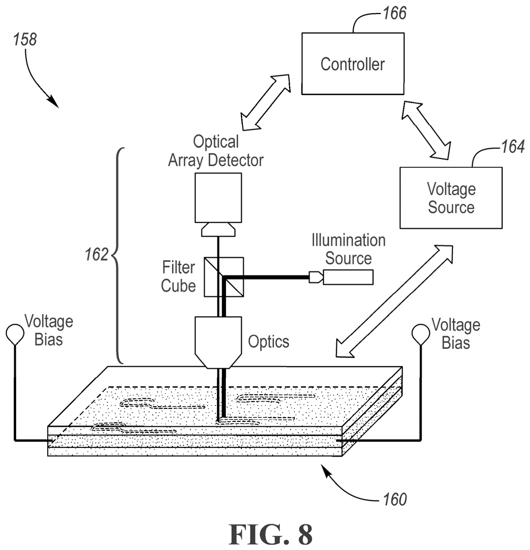

A system for optical imaging of a labeled vertex trapped polynucleotide strand for the purpose of determining a sequence of the polynucleotide strand is also provided herein. Figure 8 illustrates a system 158 according to an embodiment. The system 158 may include a device 160, at least one detector 162, a voltage source 164, and a controller 166. The device 160 may include one or more reservoirs. Any of the one or more reservoirs may be sized to receive a sample including the polynucleotide strand. Any of the one or more reservoirs may be sized to receive at least one electrode. The electrode or electrodes (either alone in a reservoir, or in a reservoir along with the sample including the polynucleotide strand) may be metal, a metal alloy, or a semiconductor material for example. In at least some embodiments, the electrode may be a platinum electrode. The electrode may be carbon. For example, the electrode may be single or multi-layered graphene. The electrode may be graphite. The electrode may also be an inkjet-printed carbon deposited lead. Alternatively, the electrode may be gold, silver, titanium, palladium, copper, stainless steel, titanium nitride, or silver/silver chloride. The electrode may also be fabricated from doped silicon. The device may also include a chip. The chip may be a microfluidic chip. The chip may be a silicon substrate chip. The chip may have two isolated (independent) single-input, single-output channels. The channels may be fabricated so that they can be visualized simultaneously within the same field of view near the geometric center of the silicon chip. The microfluidic channels may be dry-etched into the silicon wafer to a depth of 1 $\mu$m for example. The microfluidic channels may be in fluidic contact with the reservoir or reservoirs. The device may also include a channel cover. The channel cover may be glass. The channel cover may for example be borosilicate glass.

The system may also include at least one detector. An optical detector configured to visualize the trapped polynucleotide strands in the channel may be included. For example, the optical detector may be an epifluorescence microscope system. The epifluorescence microscope system may include a water immersion lens, an illumination source, and at least one camera.

The system may also include a voltage source for applying the electric field to the polynucleotide strand in the channel. The voltage source may apply a voltage using the electrodes in the reservoirs. The voltage source may also measure current.

The system may also include a controller. The controller may include a voltage source component. The controller may be programmed to interact with the device and/or pumps or flow component devices outside or within the device. The control may also be programmed to interact with the at least one detector and a separate voltage source if included. For example, the controller may direct the flow of a polymer to the device to promote delivery of the polymer to the surface of the channel so that the polymer is capable of physically interacting with the surface of the channel. The controller may similarly direct the flow of the sample including the labeled polynucleotide strand with or without the polymer to the device to promote delivery of the sample to the surface of the channel. The controller may additionally be programmed to interact with the at least one detector. For example, the controller may be programmed to receive, record and/or store signals obtained by the at least one detector. The controller may also be programmed to process the signals obtained by the at least one detector or from other sensors integrated into the system. The other sensors may be within the fluidic device or outside of it.

EXAMPLES

The following examples illustrate the various embodiments of the present disclosure. Those skilled in the art will recognize many variations that are within the spirit and scope of the present disclosure.

*Example 1: Trapping long single-molecule DNA strands*

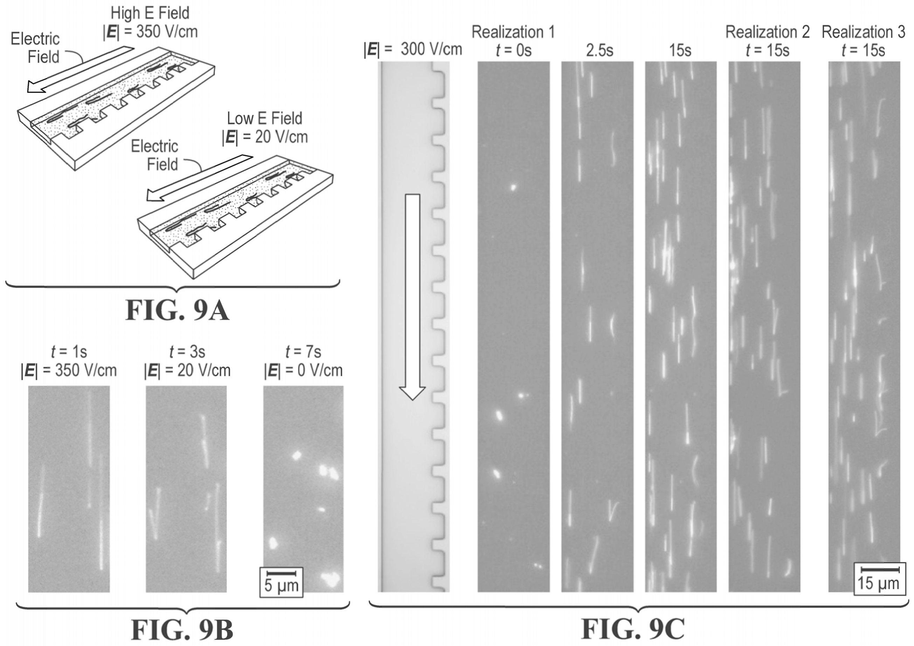

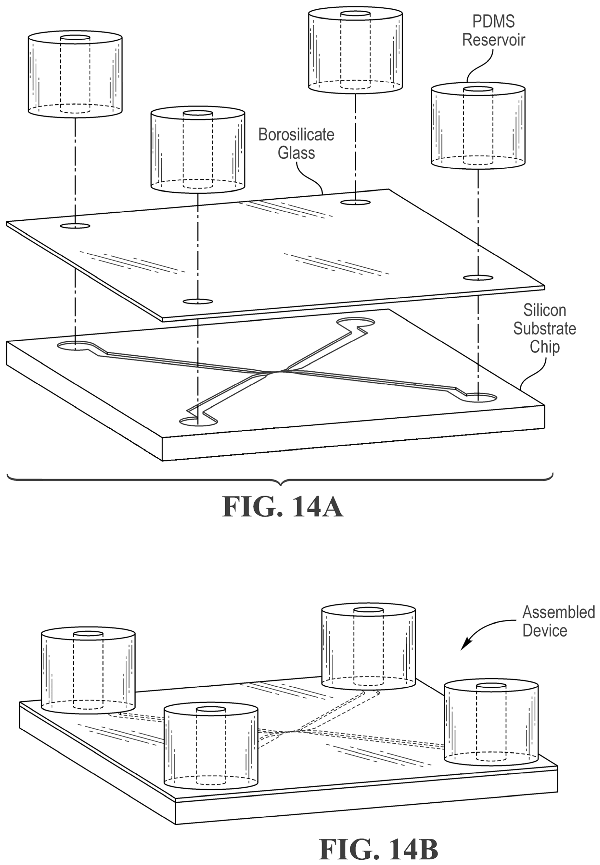

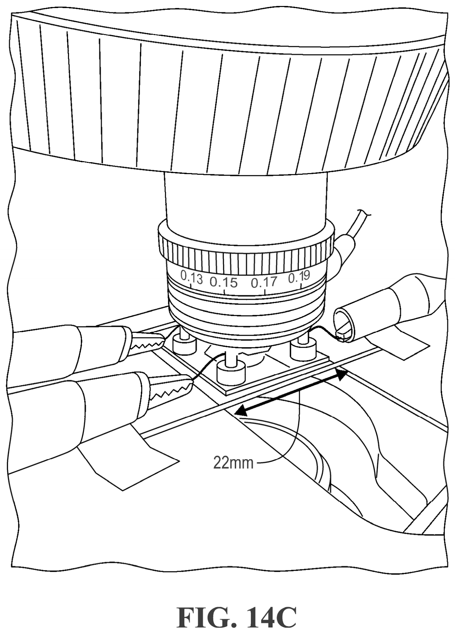

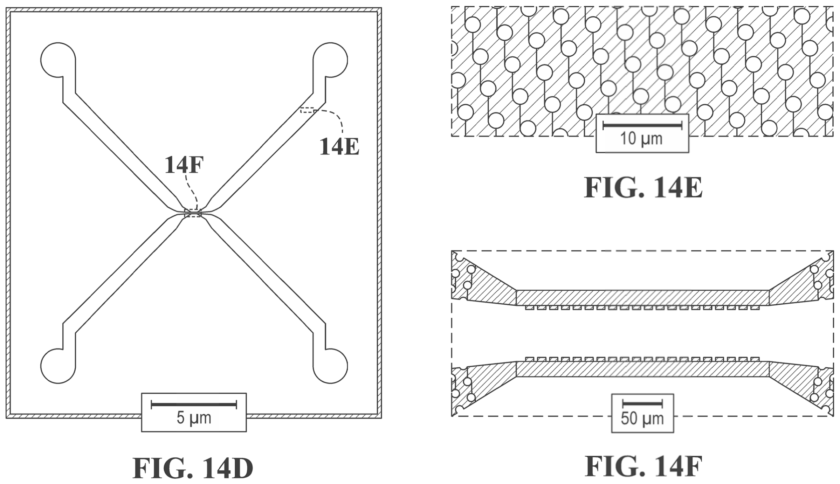

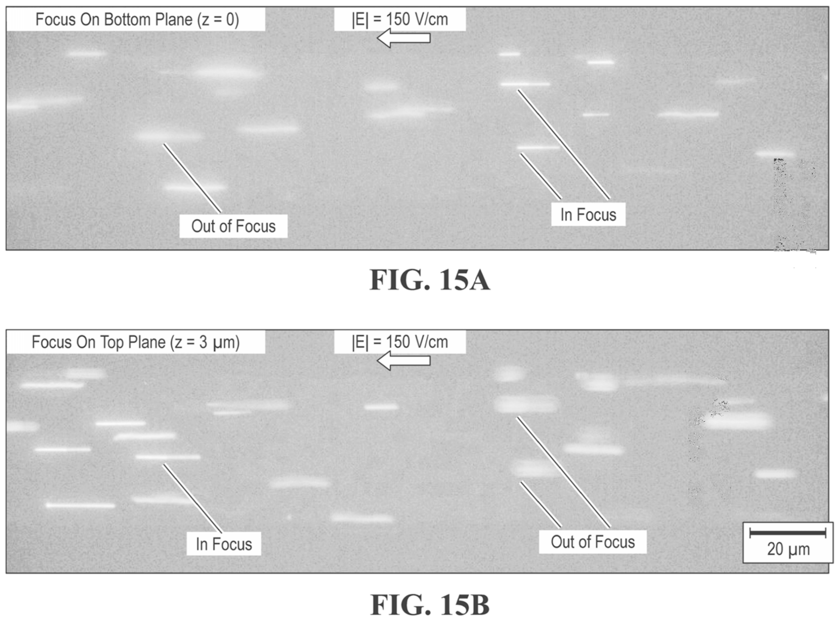

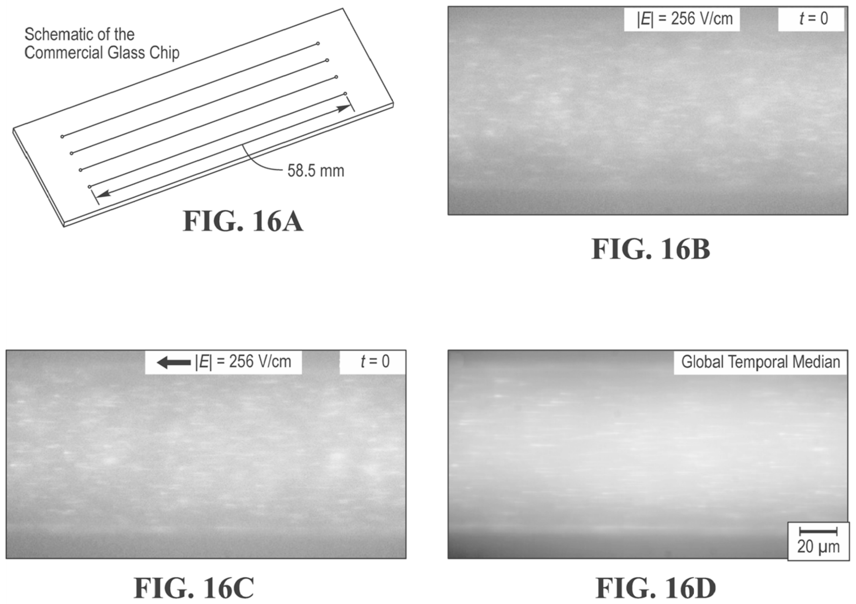

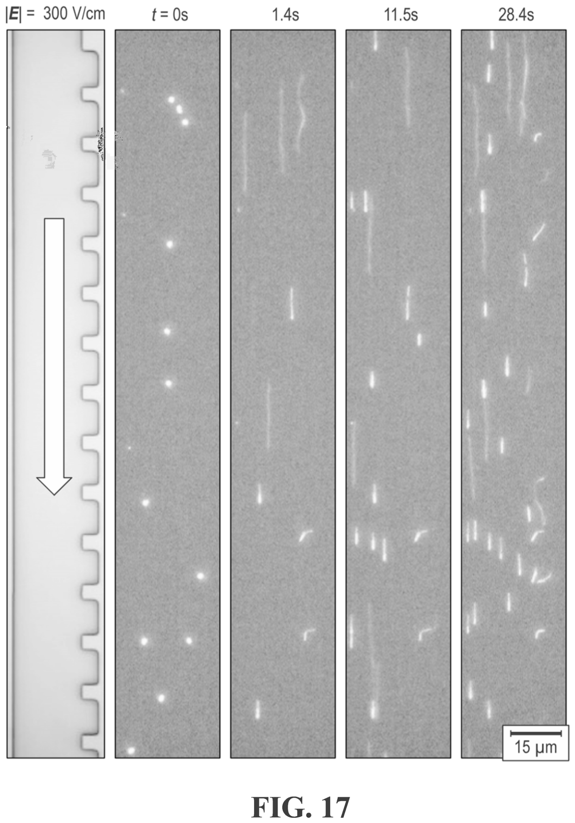

Figures 9A-9C illustrate the method of DNA trapping according to an embodiment. In this example, vertex-pinned, single-molecule 48 kbp DNA is shown in a microfluidic channel filled with a linear polymer buffer solution and subject to an applied axial electric field. Figure 9A illustrates isometric schematics of DNA pinned at a vertex with two arms extending opposite to the applied field. Figure 9B illustrates experimental epifluorescence images of vertex-pinned single-molecule DNA. Images show stretching at 350 V/cm, 20 V/cm, and no applied electric field where DNA relaxes to a Brownian coil around the vertex. Figure 9C illustrates three consecutive images of single-molecule DNA pinning upon application of 300 V/cm. The fourth and fifth images are two additional realizations of the trapping process. Solution was 4 pM of YOYO-1 labeled $\lambda$-DNA in 1$\times$TBE buffer with 2% w/w 1300 kDa PVP and 2% v/v $\beta$-mercaptoethanol. The channel has a 0.9 $\mu$m depth and a nominal spanwise width of 30 $\mu$m (see Fig. 14 for more details). The channel includes a castellation pattern on one side which is not required for the trapping (see Fig. 15 and Fig. 16 for further discussion). Figure 9A shows schematics of each DNA pinned at the wall at a vertex with two loose arms stretching in the direction opposite the applied electric field, consistent with the electrostatic force on the polymer and opposing any electroosmotic flow (EOF). Figure 9B shows consecutive false-color epifluorescence images of single-molecule 48.5 kbp DNA at electric fields of 350, 20, and 0 V/cm. First, DNA traps at high field. Upon lowering the field, the DNA trapping persists and DNA arms overlap to a lesser degree due to Brownian motion. Electric field was deactivated at t = 3.5 s, and DNA molecules coil into a three-dimensional (3D) cloud centered around the aforementioned vertex pin point (at t = 7 s). The leftmost panel of Figure 9C shows a bright-field image of the channel geometry. The figure also shows three consecutive images capturing the accumulation of vertex-pinned, single-molecule DNA. The process may be cycled, enabling high-throughput visualizations of many DNA in the same field of view. The last two images of Figure 9C show two subsequent realizations of the trapping process. Similar data for 20 kbp DNA is provided in the SM (c.f. figure 17).

Experiments were performed at electric fields ranging from 19 to 455 V/cm and at a variety of buffer chemistries, including with and without linear polymer additives typically used to suppress electroosmotic flow. DNA trapping was observed via vertex pinning only at sufficiently high electric fields and only in the presence of polyvinylpyrrolidone (PVP) with a molecular weight (MW) of 360 kDa or larger. In one or more embodiments, the PVP polymer at least partially adsorbs to glass and/or oxide channel walls thereby increasing the local solution bulk viscosity within the electric double layer. The individual DNA molecules may become entangled with one or more adsorbed PVP molecules and this entanglement most often occurs at a single point along the DNA, resulting in the configuration described in Fig. 9. The pinning occurs on the silicon oxide wall of the channels and on the surface of the glass wall used to seal the channels (see Fig. 15). Figures 15A and 15B illustrate DNA vertex-pinning at bottom and top surface of a 3 $\mu m$ deep straight channel under axial electric field strength of 150 V/cm. Figure 15A shows vertex-pinned DNA at the bottom surface, which is thermally grown thermal oxide. Figure 15B shows vertex-pinned DNA top surface, which is anodically bonded borosilicate glass. Figures 15A and 15B illustrate two consecutive images from one realization. The depth of focus is estimated to be 0.7 $\mu m$.

Figures 16A through 16D illustrate vertex-pinned, single-molecule DNA in a 37 $\mu m$ deep commercial glass microfluidic channel filled with a linear polymer solution and subject an axial electric field. Figure 16A illustrates a schematic of the commercial glass chip purchased from Microfluidic ChipShop. On each chip, four 58.5 mm long parallel glass channels are fabricated and sealed with 210 $\mu m$ lids. All materials are glass. Figures 16B and 16C illustrate consecutive epifluorescence raw images of DNA electromigrating through a 37 $\mu m$ deep commercial glass channel. The depth of field is estimated to be around 1 $\mu m$, and most DNA are out of focus. In the background near to the wall surface, vertex-pinned DNA can be observed. Figure 16D illustrates a global temporal median of the image sequence. The image amplifies the vertex-pinned DNA at the wall.

Figure 17 illustrates vertex-pinned, single-molecule 20 kbp DNA in a 0.9 $\mu m$ deep microfluidic channel filled with a linear polymer buffer solution and subject to an applied axial electric field. Wide-field, consecutive raw images of single-molecule DNA pinning upon application of 254 V/cm. Solution was 4 pM of YOYO-1 labeled 20 kbp DNA in 1×TBE buffer with 2% w/w 1300 kDa PVP and 2% v/v $\beta$-mercaptoethanol. Raw images are shown for $t$ = 0, 1.4, 11.5 and 28.4 s. The faint lines in the background are due to streaking of 20 kbp DNA under fast electromigration.

*Example 2: Testing the effects of the electric field on DNA trapping (Experimental quantification of the amount of vertex-pinned, single-molecule DNA as a function of field strength)*

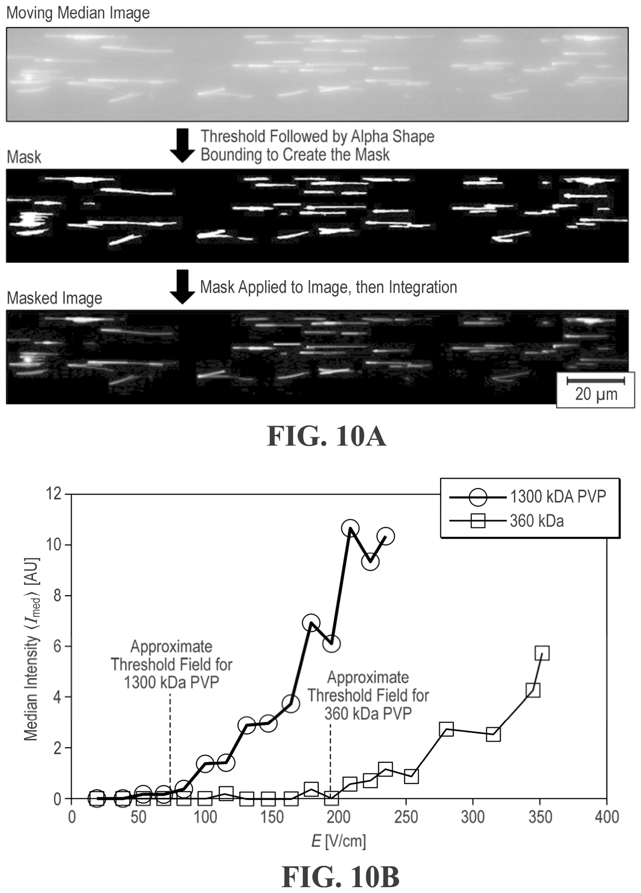

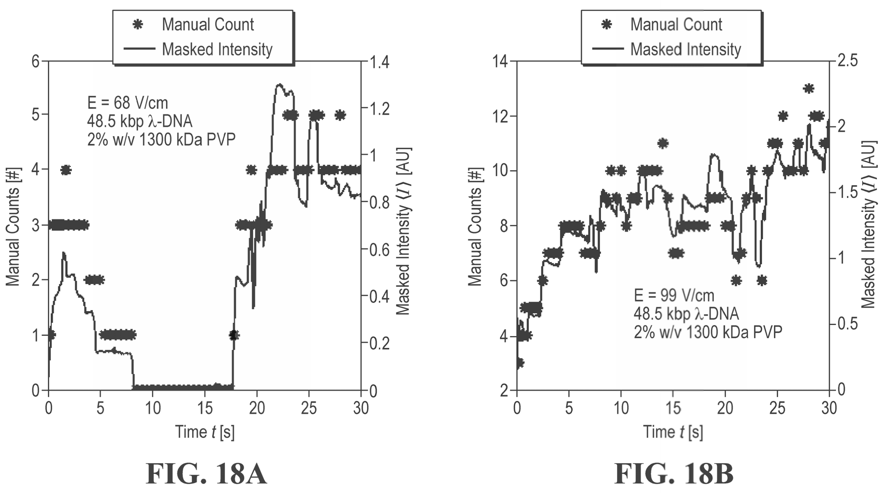

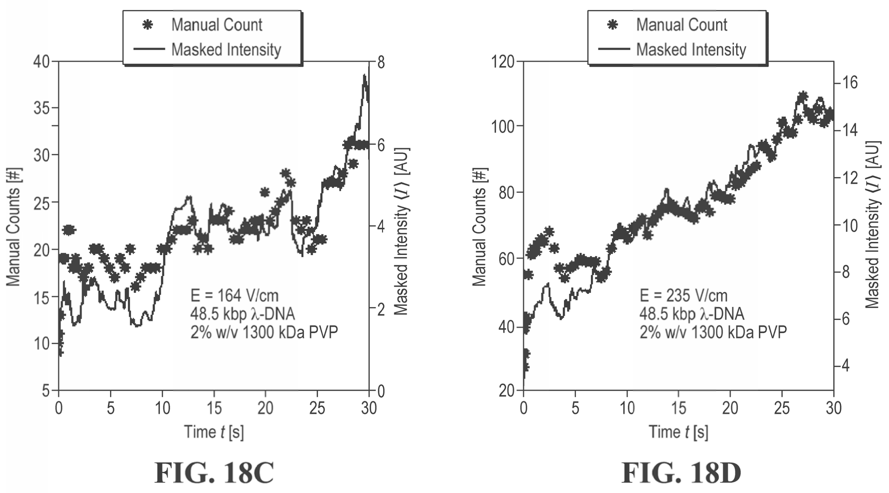

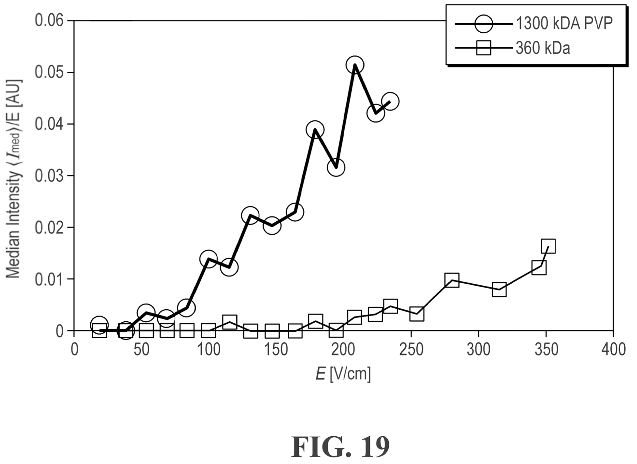

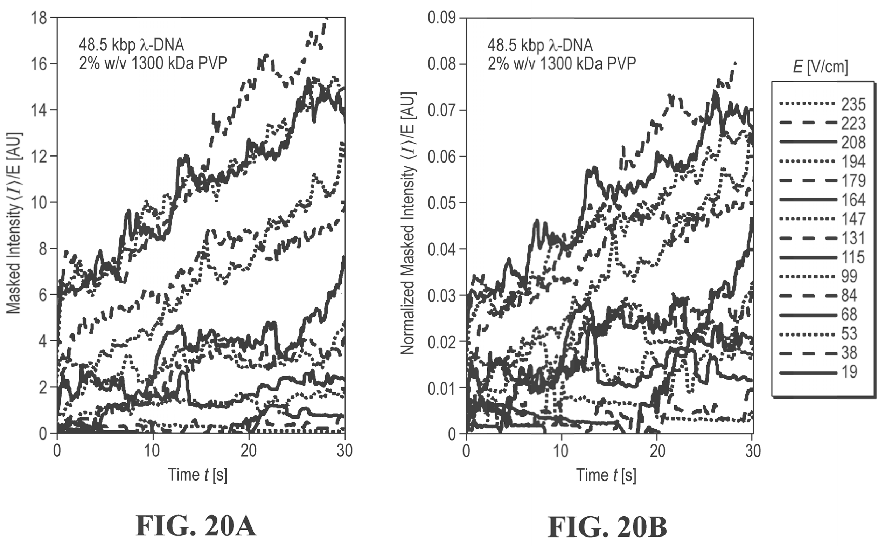

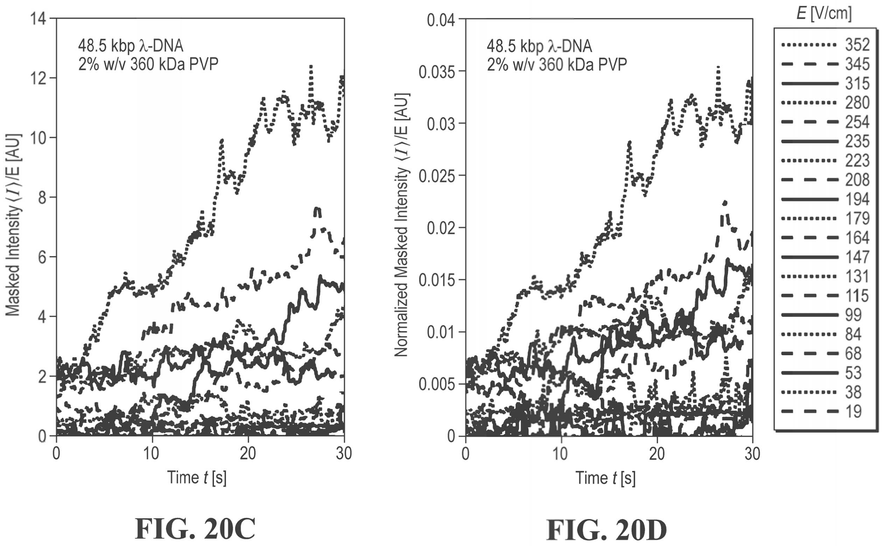

The effects of applied electric field magnitude, E, on the initiation and rate of DNA trapping was explored. A custom image processing algorithm was developed and used to quantify the number of DNA pinned as a function of E. Figure 10A summarizes this analysis and additional details are provided. Fig. 10A illustrates a flow chart summary of image processing used to quantify the amount of DNA trapping. Medians of small numbers of images were subsequently adaptively thresholded. The binarized images were used to create alpha shape bounding masks, and raw image data integrated within the regions highlighted by binarized masks. Fig. 10B illustrates area-averaged temporal median intensity, ⟨$I\_med$⟩, for 30 s duration experiments at each applied E. All data are for 4 pM of YOYO-1 labeled λ-DNA (48.5 kbp) in 1×TBE buffer with 2% v/v β-mercaptoethanol. The dark gray curve with circle markers and the light gray curve with square markers show experiments using solutions prepared with PVP MWs of MWs of 1300 or 360 kDa, respectively (both 2% w/w concentration). Dashed vertical lines highlight approximate threshold fields observed for the initiation of DNA trapping. Briefly, moving median images were computed from small groups of images to enhance signal from stationary DNA (3-image sequences for the largest E and 21-image sequences for the lowest E). Adaptive, local thresholding was then applied to obtain a binarized image. The binarized image was used as the input for an alpha-shape algorithm followed by dilation and erosion to obtain an alpha shape bounding mask. This mask was applied to the aforementioned moving median image sequences and the product integrated to obtain a scalar measure (versus time) of stationary DNA for each E. In Figure 18, this scalar correlates well with example manual counts of trapped DNA (obtained by analyzing each frame manually). Figures 18A through 18D illustrate a comparison between area-averaged alphashape-bounded intensity (*I*) versus results from manual counting of molecules. Curves show the alpha shape bounding intensity versus time for electric field strength of 18, 99, 164, and 235 V/cm, respectively. Scatter points show the manual counting of each entangled DNA at the corresponding time step for electric field strength of 18, 99, 164 and 235 V/cm, respectively. All data are 4 pM of YOYO-1 labeled λ-DNA (48.5 kbp) in 1×TBE buffer with 2% v/v $\beta$-mercaptoethanol added with 1300 kDa PVP polymer. The alpha shape bounding intensity versus time have trends that are similar to the manual counting results. We computed the median of the scalar time-series data from the automated analysis over 30 s durations for each E. Figure 10B shows these median scalar measures as a function of E for 4 pM of YOYO-1 labeled λ-DNA in 1×TBE buffer with 2% β-mercaptoethanol. Shown are data for 2% w/w PVP with MW of 1300 (curve with circle markers) or 360 kDa (curve with square markers). Each of the E field data (for 360 and 1300 kDa) exhibited an approximate threshold E value required to initiate trapping (see also Fig. 19 and Fig. 20). The observed thresholds were 70 V/cm for the 1300 kDa PVP and 180 V/cm for the 360 kDa PVP. We also performed experiments using buffers with 2% w/w PVP with MWs of 10 and 58 kDa PVP. No DNA trapping was observed for solutions prepared using these lower, commonly used, commercially available PVP weights. Referring again to the aforementioned PVP entanglement hypothesis, these observations suggest that sufficiently long PVP is required for PVP to adsorb to the surface of the channel and present a PVP scaffold that enables DNA entanglement onto PVP.

Figure 19 illustrates an experimental quantification of the amount of vertex-pinned, single-molecule DNA as a function of field strength. Median intensity of each realization of applying electric field for 30 s are plotted on the ordinate versus the axial electric field strength of each realization on the absicca. Different from Figure 10B, here the intensity is normalized by the applied axial field strength. All data are 4 pM of YOYO-1 labeled $\lambda$-DNA (48.5 kbp) in 1×TBE buffer with 2% v/v $\beta$-mercaptoethanol. The curve with circle markers and the curve with square markers show experiments of using sample of adding 2% w/w concentration of PVP with MWs of 1300 and 360 kDa, respectively.

Figures 20A through 20D illustrate an experimental quantification of the amount of vertex-pinned, single-molecule DNA as a function of time and field strength. Alpha shape bounding masked intensity versus time are shown for realizations performed at multiple respective electric fields. All data were obtained from 4 pM of YOYO-1 labeled $\lambda$-DNA (48.5 kbp) in 1×TBE buffer with 2% v/v $\beta$-mercaptoethanol. Figures 20A and 20B have an addition of 2% w/w 1300 kDa PVP polymer in the buffer and Figures 20C and 20D have an addition of 2% w/w 360 kDa PVP polymer in the buffer. Intensity in Figure 20B and 20D are normalized by the applied axial electric field strength for each realization.

The data suggests that a high magnitude electric field is required for high friction force between the DNA and PVP polymers. Given the threshold nature of the required field, a sufficiently high electric force on the DNA may be required to expel a hydration layer between the DNA and PVP molecules. Elimination of a hydration layer may increase the solid friction between charged DNA polymer and the uncharged linear PVP polymer. This may lead to the observed persistent, high-friction entanglement at a fixed vertex. This hypothesis is consistent with the fact that persistent pinning can occur at a vertex near the end of a DNA molecule such that the two loose arms have widely disparate lengths and so experience much different forces. The elimination of hydration layers between single DNA molecules and polymer molecules has been implicated in studies of DNA interacting with agarose cross-linked polymer. In studies of DNA electrophoresis through 3D agarose gel network, it was found that DNA can be trapped under high electric fields. Another study observed trapping of DNA within an agarose gel for electric fields above certain threshold of electric fields and proposed the "knot" trapping hypothesis. However, the entangled DNA in these previously studies acquired a complex 3D shape within the interior of the gel and not a "clean" vertex pinning at a wall with two straight arms (within a linear polymer solution) as in the current work.

*Example 3: Testing relaxation dynamics of vertex-pinned DNA*

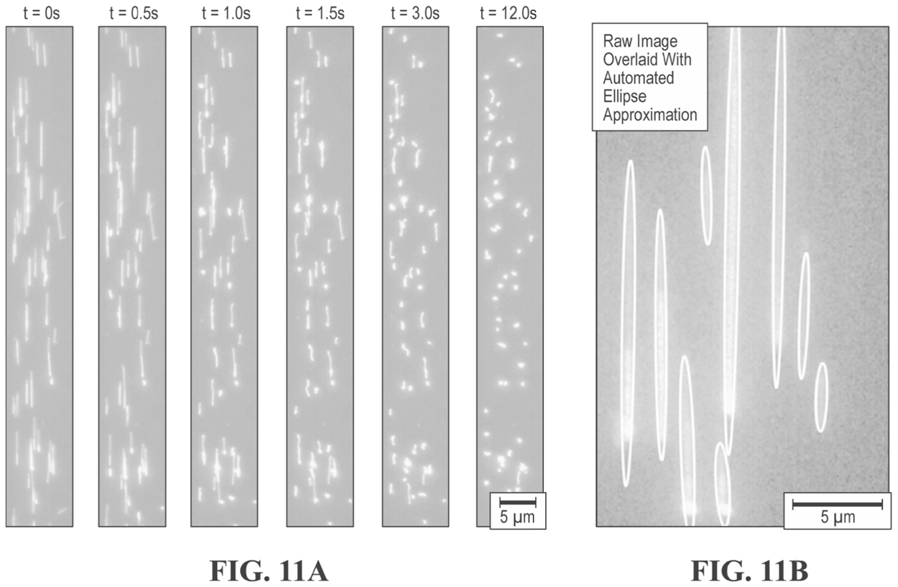

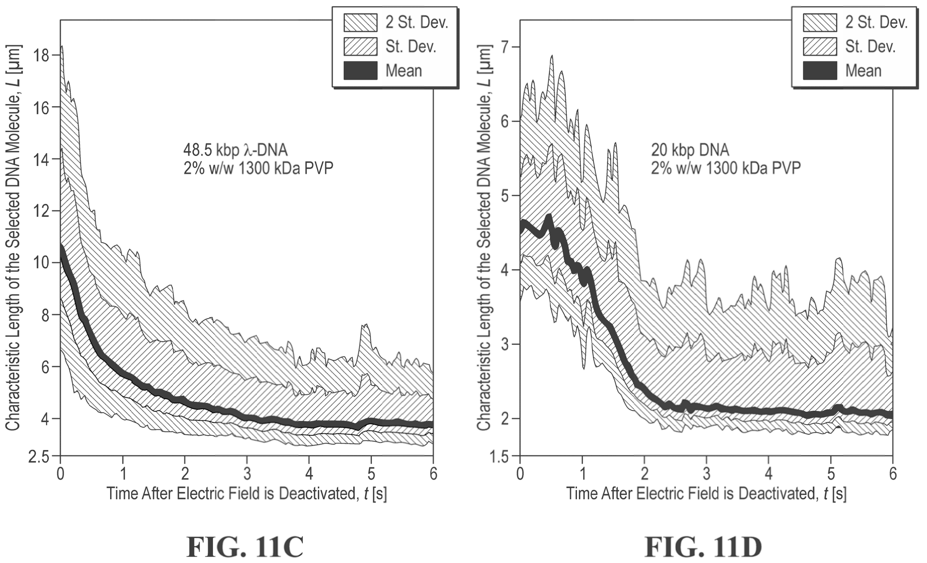

The relaxation dynamics of vertex-pinned DNA upon removal of the electric field were analyzed. Figure 11A shows a time-sequence of images. We observed DNA pinned at random locations along the length of the DNA molecule, and this led to a range of relaxation timescales. We adopted an automated image processing method based on adaptive thresholding, fitting of DNA images with an ellipse, extracting of the major and minor axes of the best-fit ellipses. Figure 11B shows a close-up image with best-fit ellipses. More specifically, Fig. 11A illustrates example sequence of raw epifluorescence microscopy images of pinned $\lambda$-DNA (48.5 kbp) immediately after deactivation of the applied electric field (at $t = 0$ s). Fig. 11B illustrates example raw image with superposed best-fit ellipses used to quantify DNA length and width. Figures 11C and 11D show plots of the statistics of ellipse major axis lengths, $L$, for relaxation of 48.5 and 20 kbp DNA molecules versus the time since deactivation of the field. Black curve shows the instantaneous mean of $L$. The dark gray and light gray shaded curves show one and two standard deviations of the distributions, respectively.

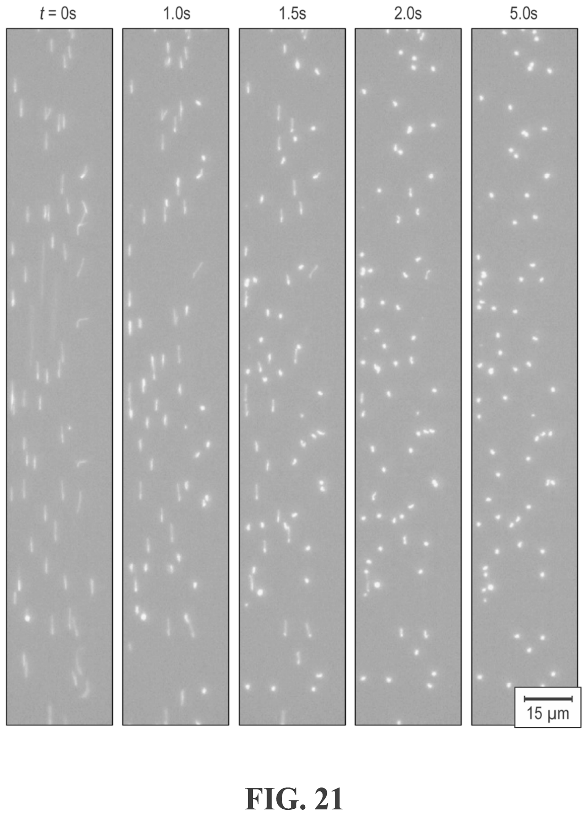

Figures 11C and 11D show statistical moments of characteristic lengths of pinned DNA molecules as they relax. Figures 11C and 11D show data for $\lambda$- (48.5 kbp) or 20 kbp DNA, respectively, labeled with YOYO-1 dye in 1×TBE buffer of 2% v/v $\beta$-mercaptoethanol and 2% w/w PVP of MWs of 1300 kDa, respectively. The initial mean values for L were lower than the expected full contour lengths as most DNA were pinned at a vertex well away from the end of the molecule. Vertex-pinned 48.5 kbp DNA molecules took approximately 8 s to fully relax, while vertex-pinned 20 kbp DNA took approximately 3 s to fully relax. Epi-fluorescence images of relaxation of vertex-pinned 20 kbp DNA are included in Fig. 21. Figure 21 illustrates the relaxation dynamics of vertex-pinned, single-molecule DNA. Example sequence of raw epifluorescence microscopy images of pinned 20 kbp DNA immediately after deactivation of the applied electric field (at $t = 0$ s).

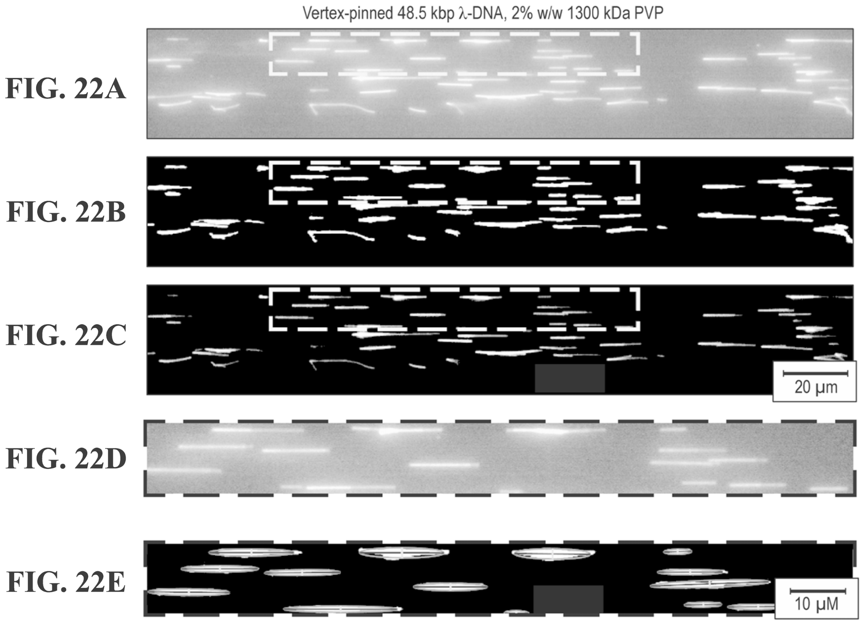

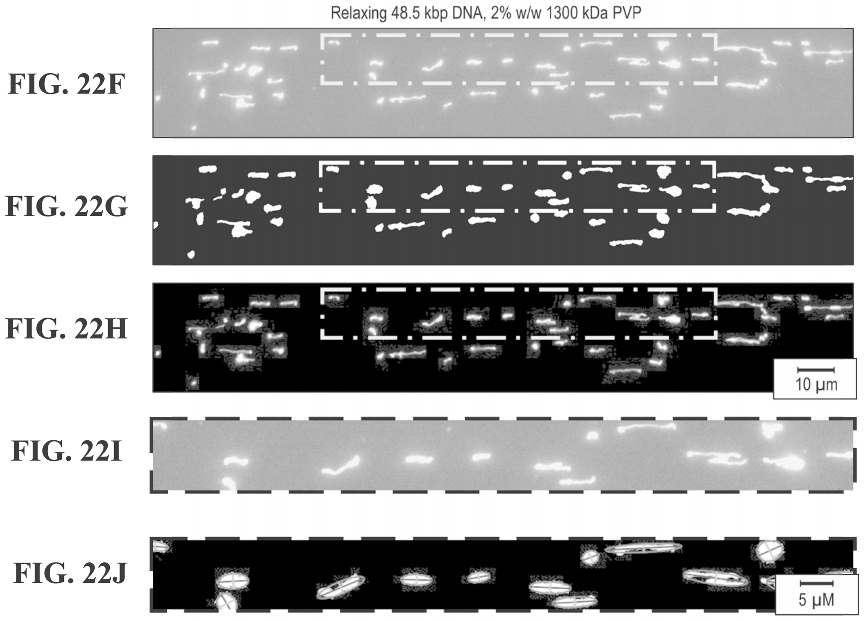

Figures 22A through 2J illustrate a summary of automated image processing used to quantify 48.5 kbp DNA relaxation. Figure 22A shows an example image of vertex-pinned DNA before relaxation. Figure 22B shows a binarized image of Figure 22A after adaptive thresholding and alpha shape operation. The image in Figure 22C was obtained by integrating Figure 22A with the mask in Figure 22B. Figure 22D illustrates a zoomed in image of Figure 22A. Figure 22E illustrates an example image to show the automated detection of each island. The ellipse curve shows the result of ellipse shape approximation. The black lines and the light gray lines within the ellipses show the major and secondary axis of each approximated ellipse shape, respectively. Figures 22F through 22J illustrate images showing the same procedure as from Figures 22A to 22E, except that the image was chosen during the DNA relaxation process.

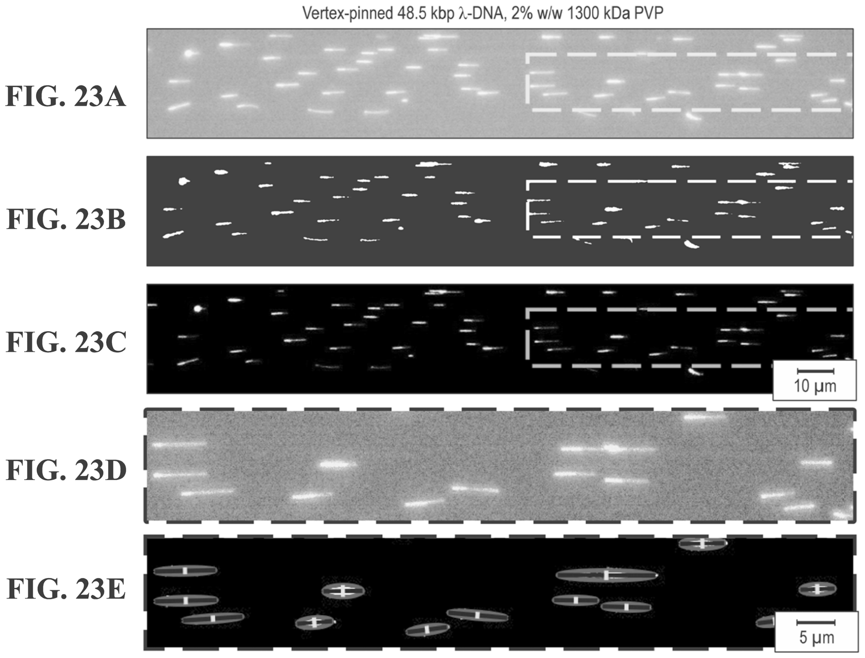

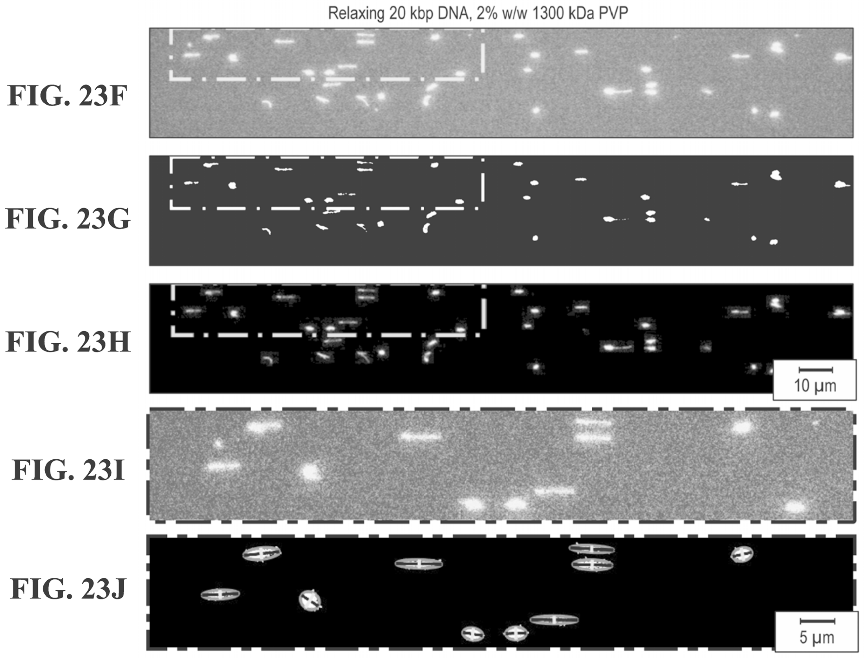

Figures 23A through 23J illustrate a summary of automated image processing used to quantify 20 kbp DNA relaxation. Figure 3A illustrates an example image of vertex-pinned DNA before relaxation. Figure 23B illustrates a binarized image of Figure 23A after adaptive thresholding and alpha shape operation. The image in Figure 23C was obtained by integrating the image of Figure 23A with the mask in Figure 23B. Figure 23D illustrates a zoomed in image of Figure 23A. Figure 23E illustrates an example image to show the automated detection of each island. The ellipse curve shows the result of ellipse shape approximation. The black lines and the light gray lines show the major and secondary axis for each approximated ellipse shape, respectively. Figures 23F through 23J illustrate images showing the same procedure as from Figures 23A to 23E, except that the image was chosen during the DNA relaxation process.

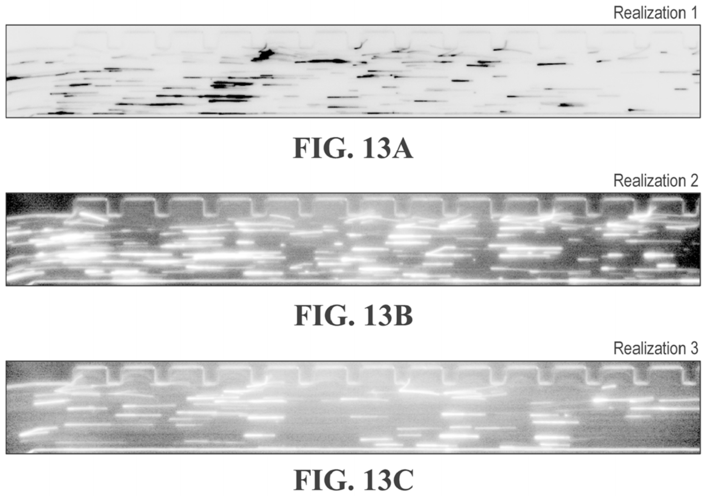

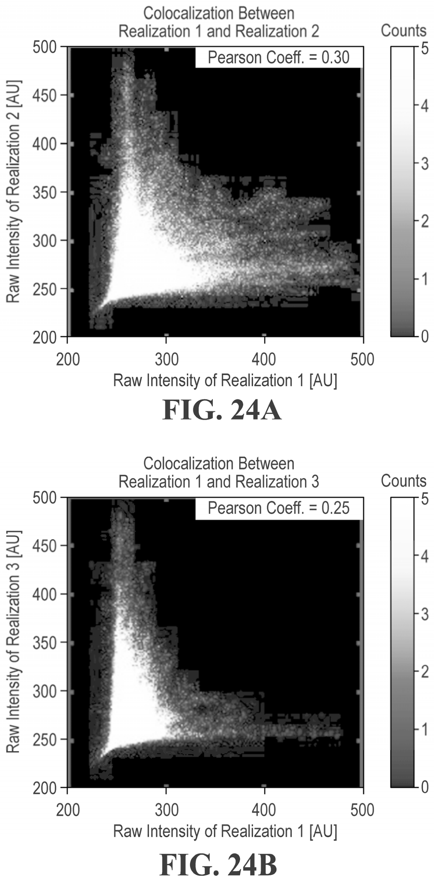

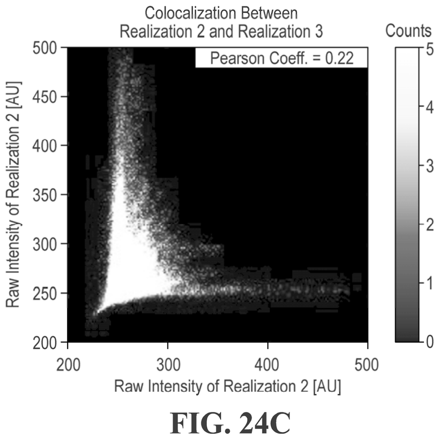

Figures 24A through 24C illustrate a colocalization analysis of image intensity of vertex-pinned DNA across three different realizations. Colocalization data are shown for three pairs of independent realizations of DNA trapping. Realizations 1, 2 and 3 are DNA vertex-pinned DNA data from Fig. 13A, 13B and 13C in the main text, respectively. Figures 24A through 24C illustrate colocalization scatter plots (correlation diagrams) are shown comparing each pair of realizations. As indicated by the intensity bar on the right, the heat map are the counts based on raw intensity values on the collocated pixels from two images of two realizations. Pearson correlation coefficients are computed between each pair, and the values are 0.30, 0.25 and 0.22, respectively.

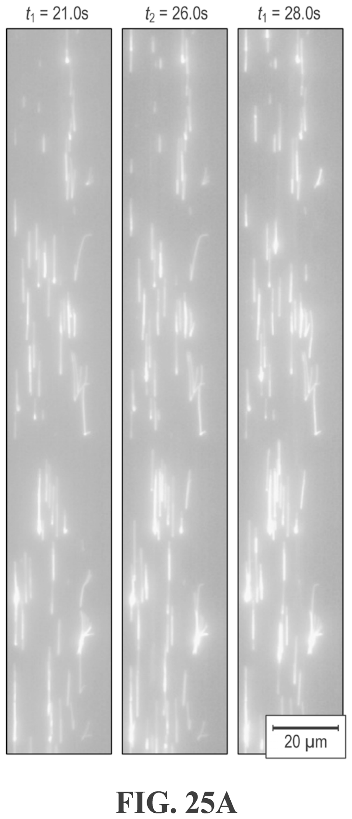

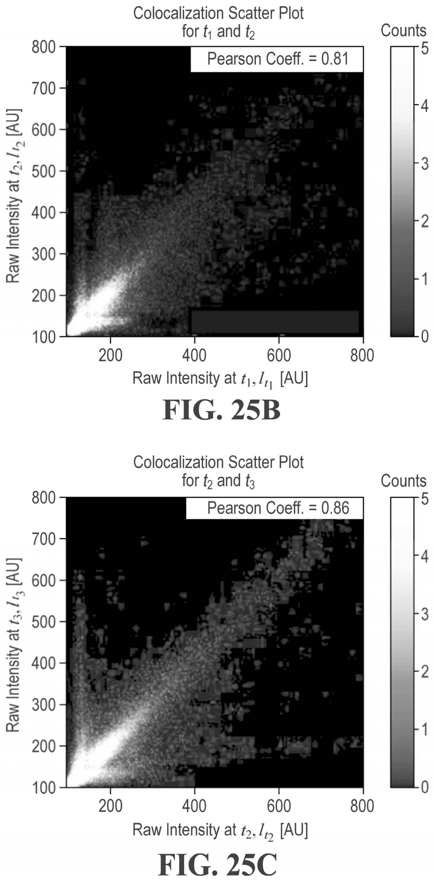

Figures 25A through C illustrate a colocalization analysis of image intensity of vertex-pinned DNA across three consecutive images from one realization. Figure 25A illustrates three raw consecutive epi-fluorescence images are shown for a single realization of DNA vertex-pinning experiments. The first sub-panel and the second sub-panel are 5 s apart. The second and third subpanels are 2 s apart. Figures 25B and 25C illustrate colocalization scatter plots (correlation diagrams) comparing each consecutive image pair. The computed Pearson correlation coefficients values for the two time-sequenced image pairs are 0.81 and 0.86, respectively.

As described above, upon removal of the (high) electric fields, the DNA consistently relaxes toward and around the vertex of the pinned location. This further supports a hypothesis of a single-point entanglement between DNA and PVP. The persistence of such a pinning point (for order 30 s) suggests that the observed DNA trapping is not due to electrosorption forces such dielectrophoretic forces which may manifest from wall imperfections (e.g., roughness elements). Such electrosorbed DNA molecules would be expected to quickly "release" upon deactivation of the field. In this example, the DNA were not trapped at the convex corners of castellation features in the channel.

*Example 4: Testing cycling of DNA trapping, relaxation, and disentanglement*

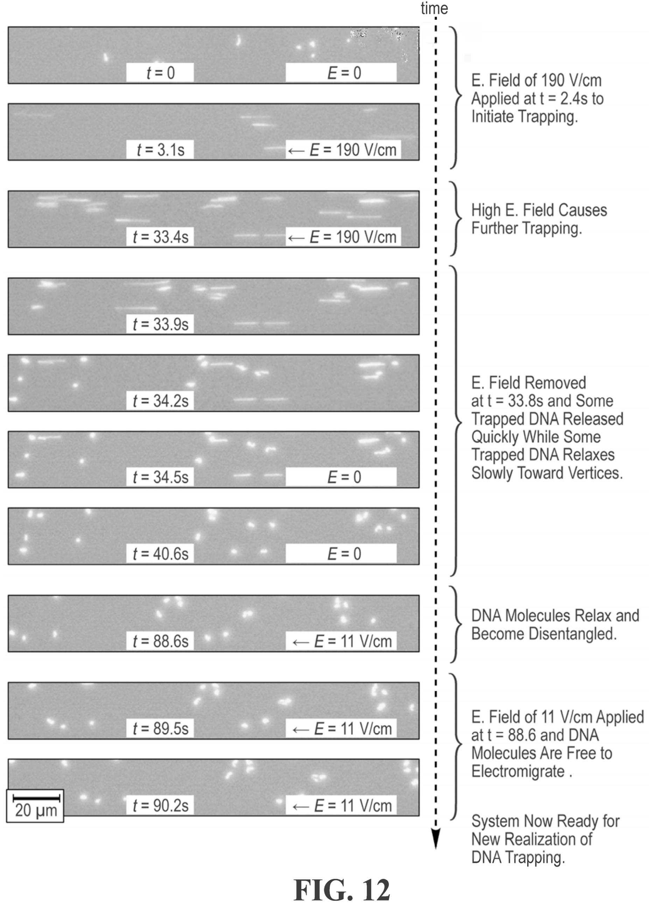

Figure 12 shows a representative cycle of DNA vertex-pinning at high electric field; relaxation upon deactivation of field; disentanglement after about 36 s; and then a state of free diffusion and electromigration. DNA molecules were initially in a three-dimensional Brownian coil at t = 0 s. An electric field of 190 V/cm was activated at t = 2.4 s to initiate trapping. The electric field was deactivated at t = 33.8 s and DNA molecules quickly relaxed toward their vertex/pin point (c.f. Fig. 11). Over times longer than about 50 s, relaxed DNA become unpinned from the wall and were again free to diffuse and electromigrate. At t = 88.6 s, a low axial electric field of 19 V/cm was applied to clear the field of view (FOV) of previously pinned DNA. The process can be repeated in multiple, subsequent cycles with an estimated period of order 60 s. Figure 12 illustrates consecutive (top to bottom) images of single-molecule DNA. DNA molecules are initially in expected random coil shapes. E = 190 V/cm was applied at *t* = 2.4 s to initiate vertex pinning. E deactivated at *t* = 33.8 s, DNA molecules quickly released or slowly relaxed towards the vertices. After about 50 s, more than 95% of DNA became unpinned from the wall. At t = 88.6 s, a relatively low axial electric field of 11 V/cm was applied to electromigrate away previously vertex-pinned DNA molecules. Note the displacements of correlated patterns across the last three images.

The data of Fig. 12 further support entanglement between DNA and PVP. After removal of the electric field, the space between the DNA and PVP may become re-hydrated, and the associated lower-friction configuration and Brownian motion promote a disentanglement after several 10's of seconds. A pulse of pressure driven flow may cause much faster disentanglement down to an order of a few seconds.

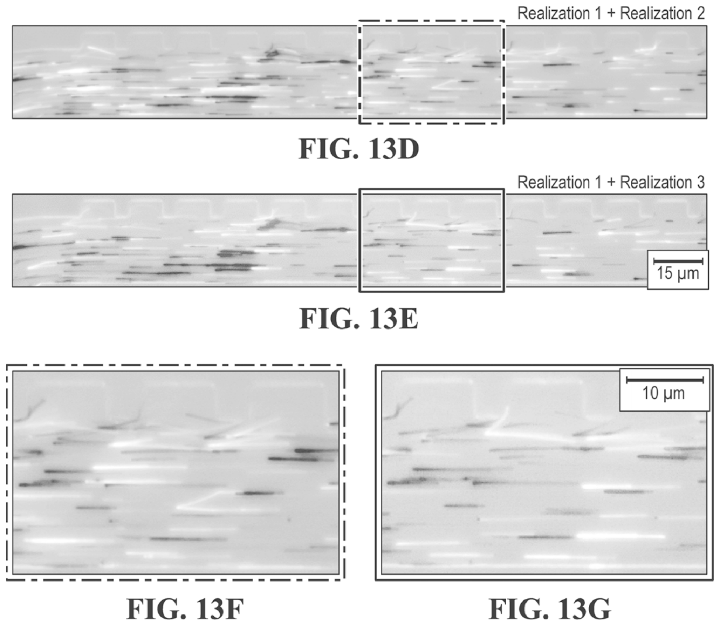

Whether DNA pinning sites are spatially correlated across realizations was also investigated. Figures 13A through 13G show that single DNA molecules are vertex-pinned to walls at locations which vary randomly from realization to realization. Figures 13A-13C show three representative realizations. The first is shown with inverted grey scale and the other two are raw greyscale images. Between realizations, DNA were allowed to fully relax and disentangle. Figure 13D shows superposition of Realization 1 and 2 images; and Figure 13E shows superposition of images of Realizations 1 and 3. The zoomed-in images of Figs. 13D and 13E are shown in Figure 13F and 13G. All data shown in Figures 13A through 13G were obtained from 4 pM of YOYO-1 labeled λ-DNA with 2% w/w 1300 kDa PVP. Figures 13A through 13C illustrate images of three separate realizations of DNA trapping using inversed grayscale (Realization 1), grayscale (Realization 2) and grayscale (Realization 3) schemes, respectively. DNA were trapped of 300 V/cm for 15 s. DNA were allowed to relax and disentangle between experiments (c.f. Fig. 4), and a low electric field was used to both confirm disentanglement and to clear previously trapped DNA. Figures 13C and 13D illustrate superposed images of Realizations 1, 2, and 3 showing poor spatial correlation of vertex capture points across two and three realizations, respectively. Figure 13F and Figure 13G are zoomed-in images of Figure 13D and Figure 13E, respectively.

We observed a poor correlation of DNA capture location among all realizations studied. This result further supports a hypothesis that the observed trapping is not due to specific features in the channel geometry such as roughness elements or nano-scale surface pits. The data supports that DNA molecules become entangled with adsorbed PVP molecules. This entanglement is eventually reversible and new DNA molecules become pinned at new, uncorrelated locations. Approximately linear growth of the trapping rates shown in Fig. 10 suggests that the trapping sites for DNA do not reach saturation for the duration of the experiments.

*Materials and Methods for Examples 1 through 4:*

Custom microfluidic interface device

Figures 14A through 14D illustrate a custom microfluidic interface device according to an embodiment. Figure 14A illustrates pieces of PDMS reservoirs, 170 $\mu$m thick borosilicate glass and a silicon substrate chip. Figure 14B illustrates the assembled custom microfluidic device. Borosilicate glass was anodically bonded to the silicon substrate chip and then PDMS reservoirs were plasma bonded to the borosilicate glass. Figure 14C illustrates the assembled custom microfluidic device taped to a glass slide and then visualized via a water immersion 60× objective of numerical aperture of 1.2. The illumination source is a blue LED. Platinum electrodes were inserted into the reservoir filled with liquid. Figure 14D illustrates the layout of the whole silicon substrate chip and zoomed in images of two regions are shown. The center region has castellation features near one side. The large microchannel regions leading to reservoirs have posts arrays of diameter of 10 $\mu$m. The posts array is designed to support the anodically bonded borosilicate glass. More specifically, figure 14A shows the three main components of a microfluidic device of one or more embodiments which are four polydimethylsiloxane (PDMS) reservoirs, a borosilicate glass slip (channel cover), and a silicon substrate chip. Figure 14B shows an isometric view of the device after bonding and assembly. Figure 14C shows an image of the experiment showing the chip, platinum electrodes in reservoirs (connected to alligator clips), and the water immersion objective. Figure 14D shows the layout of the channels in the silicon substrate chip. The chip has two isolated (independent) single-input, single-output channels. The channels were fabricated so that they could be visualized simultaneously within the same field of view near the geometric center of the silicon chip. Fabrication was carried out on 4-inch n-type silicon wafers (resistivity of 4 Ohm-cm). Following standard photolithography, the microfluidic channels were dry-etched into the silicon wafer to a depth of 1 $\mu$m (actual resulted depth was 0.9 $\mu$m measured by profilometer) using HBr and BC13 (Oxford Instruments PlasmaLab III-V etcher). Residual photoresist was removed using oxygen plasma which was followed by a 20-minute piranha cleaning at 120 °C. 300 nm thick thermal oxide was then grown on the wafers by Rogue Valley Microdevices. The wafers were diced with a wafer saw to 9 devices each with in-plane dimensions of 22 by 24 mm. These devices were then subjected to another round of piranha cleaning. The glass cover slips were No. 1 thickness borosilicate 3.3 glass (purchased from Kemtech America Inc.). The glass drilling was performed using 1.1 diameter, triple-ripple diamond drill bits (purchased from Arrowhead Lapidary & Supple) and a manually actuated drill press. The drilled cover slips were anodically bonded. After the anodic bonding, PDMS reservoirs of 5.0 mm outer diameter (OD) and 1.5 mm inner diameter (ID) were cut using biopsy punches and then bonded to the glass using air plasma. The drilled holes in the glass were aligned with the cut holes on PDMS to form a fluidic connection.

Sample preparation

$\lambda$-DNA samples were purchased from ThermoScientific (Waltham, MA) at a stock concentration of 0.3 $\mu$g/uL. 20 kbp DNA samples were purchased from ThermoScientific and at a stock concentration of 0.5 $\mu$g/uL. YOYO-1 dye was purchased from Invitrogen at a stock concentration of 1 mM. 10× TBE buffer was purchased from Invitrogen and consisted of 1.0 M Tris, 0.9 M Boric acid, and 0.01 M EDTA. Polyvinylpyrrolidone (PVP) of molecular weights (MWs) of 10, 58, 360, and 1300 kDa were purchased from ThermoScientific. $\beta$-mercaptoethanol (MW = 78.13, >98.0% purity) was purchased from TCI America. 10×TBE buffer was initially diluted to 1×TBE buffer with molecular biology grade water (purchased from Fisher Bioreagents, Pittsburgh, PA). Next, $\beta$-mercaptoethanol was added to 1×TBE buffer to achieve 2% v/v concentration as an oxygen scavenger. PVP was then added at each aforementioned MW to the buffer at a concentration of 2% w/w. The final resulting buffers consisted of 1×TBE, 2% v/v $\beta$-mercaptoethanol, and 2% w/w of PVP with MWs of 10, 58, 360, or 1300 kDa, respectively. We measured a conductivity of 1487 $\mu$S/cm of the loading buffers via a conductivity probe (Advanced Electrochemistry Meter from ThermoScientific Inc.). The previous steps were all performed at room temperature (20±1 °C). Next, raw DNA samples were diluted in the loading buffers to achieve a final concentration of 4 pM, and YOYO-1 dye was added to achieve a base-pair-to-dye stochiometric ratio of 5:1. The samples were then incubated at 40 °C for 1 hour before use. Samples were then each incubated at 40 °C for 1 hour prior to use to facilitate dye intercalation.

Measurement apparatus

Figure 14C shows an example image of one experiment using the assembled microfluidic device. The visualizations were performed using a standard OLYMPUS BX60 upright epifluorescence microscope. We used a water-immersion 60× objective with a numerical aperture of 1.2 (OLYMPUS UPlanApo). The illumination source was a high-power blue LED from ThorLabs (SOLIS-470C) controlled with a ThorLabs DC200 controller, and the epifluorescence filter cube has the following excitation/dichroic/emission components: EX460-490, DM505, EM510IF (OLMPUS U-MWIB2). Two cameras were used for image acquisition. The first camera was a scientific complementary metal-oxide-semiconductor (sCMOS) camera manufactured by Hamamatsu (ORCA-Flash 4.0LT) which we control using HCImageLive software. The sCMOS camera was used to acquire all data in the main manuscript and the majority of the data in the Supplementary Materials (SM). The second camera was an Andor electron multiplying charge-coupled device (EMCCD) camera (iXon Ultra 897) which we controlled using Andor SOLIS software. The EMCCD camera was used to acquire image data for Figure S3 (images within a wetetched all glass channel described below).

Voltage was applied using platinum electrodes inserted at the end-channel reservoirs. The electrodes were platinum wire (0.368 mm diameter, hard, 99.95 metals basis) purchased from ThermoScientific. Voltage was sourced and current measured using a Keithley 2400 SourceMeter. The SourceMeter was triggered and controlled via RS232 interface by a personal computer (PC) using custom scripts we wrote in MATLAB (MATLAB 2023b, Mathworks Inc., Natick, MA, USA).

Image processing for quantification of amount of vertex pinned DNA

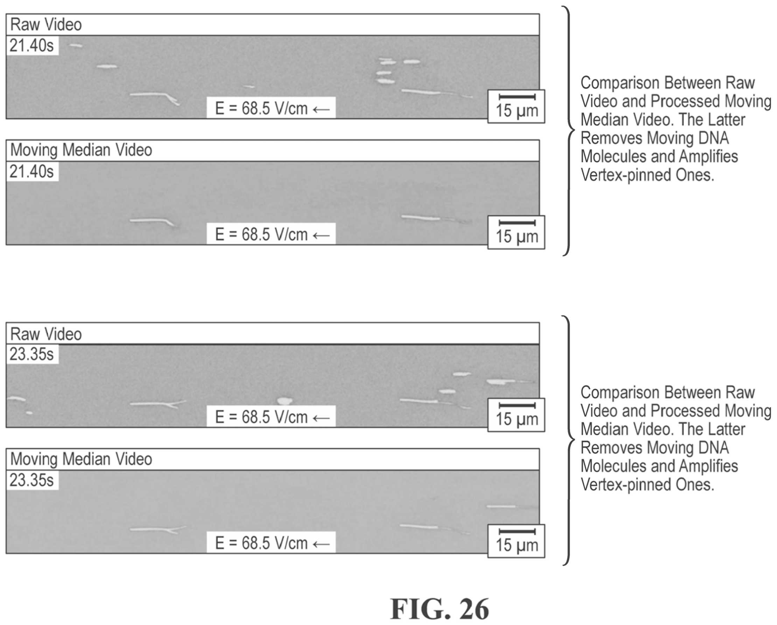

All data in the image processing section below were acquired by a Hamamatsu Orca-Flash 4.0LTsCMOS camera at 20 frame per second (fps) with an exposure time of 0.49 s. The camera has a sensor pixel size of 6.5 by 6.5 $\mu$m. The pixel size in image plane was approximately 0.11 by 0.11 $\mu$m per pixel, since we used a 60$\times$ objective without any demagnification lens. Each realization's raw TIFF sequences were imported into MATLAB for image analysis. Each 30 s sequence consisted of 600 images. First, a sequence composed of a temporally moving median operation was obtained. This moving median replaces the pixel value of each image with the median value of the same pixel across a small number of previous and subsequent images. This operation has the effect of enhancing and retaining approximately stationary image data (pinned, stretched DNA) and rejecting moving objects (electromigration DNA). The highest applied electric fields of 363 V/cm were associated with the fastest velocity. Hence, each median operation considered only a three-image group (i.e., each image pixel value was replaced by the median among the previous frame, current frame, and preceding frame). The median operation for the lowest applied electric fields (slowest moving DNA) used a progressively larger number of images within each group. A maximum of 21 frames for the lowest applied fields of 19 V/cm were considered. The top subplot of Figure 10A shows an example frame obtained from the moving median image sequence. For instance, a video taken for a comparison between raw video versus moving median video demonstrates how the image data from stationary objects (pinned DNA) are enhanced. Figure 26 illustrates example frames from this video that show the difference between raw video and the moving median video, which is processed to only show non-moving DNA molecules. Bradley's method is used to perform local mean-based adaptive thresholding on the moving median image sequences. To this end, the MATLAB command adaptthresh was used with a sensitivity parameter of 0.45 followed by imbinarize to obtain binarized images for each frame. The pixel values of the binarized images were set to zero below the threshold and to unity above the threshold. Next, the data for the thresholded pixel coordinates via the MATLAB command were identified and stored. An alpha shape operation is then performed on the binarized image to efficiently reject non-DNA image noise from the analysis. Alpha shape is a generalization of the concept of convex hull and is a subset of Delaunay triangulation. The alpha shape method is used for pattern recognition in computational graphics to identify the relatedness (via proximity) of points on a graph. To obtain the alpha shape, the binarized pixel coordinates and an alpha radius of 2.5 were used as inputs to the MATLAB command alphaShape. The resulting alpha shape was projected onto a binary mask. The binary mask was first dilated and then eroded with the same 10-pixel long streamwise line as a structuring element (again to favor even roughly horizontal, pinned DNA images). After erosion, the algorithm obtained alpha shape bounding masks. The alpha shape method successfully reduced background noise caused by adaptive thresholding. The alpha shape operation and the dilation and erosion also smoothed the noisy edges of the identified objects. The middle sub-image of Figure 10A shows an example of the resulting mask. At each time frame, the binarized mask was used on the moving median image to obtain a sequence of thresholded moving median images. The bottom sub-image of Figure 10A shows an example of the resulting operation between the alpha-shape-bonded mask and one of the median images. The (original, unchanged) intensity of detected DNA within the resulting thresholded images was then integrated spatially to acquire a scalar signal which varied with time at the frame rate of 20 fps (scalar I in Figure 11).

Image processing for analysis of relaxation of vertex-pinned DNA

This section describes the image processing analyses performed to obtain the DNA relaxation data shown in Figures 11B, 11C and 11D of the main text. First, TIFF sequences were gaussian filtered temporally with a 3-frame wide filter using MATLAB built-in function imgaussfilt3. Then, the same method explained above was followed to obtain alpha shape bounding masks for videos of DNA relaxation, except dilation followed by erosion was not included to preserve DNA shape. Once the bounding masks were obtained, the MATLAB command bwconncomp was used followed by regionprops to identify properties about distinct connected regions inside the alpha shape bounding mask. In an effort to prevent image noise and DNA fragments from remaining in the following analyses, connected regions with total intensity less than 100,000 and 20,000 for 48.5 and 20 kbp DNA sample, respectively, were removed. These thresholds were determined heuristically and designed to remove objects with significantly less intensity than largely intact pieces of DNA. Figures 21A to 21C and Figures 21F to 21H show examples of the aforementioned image processing procedures for vertex-pinned DNA before and during relaxation, respectively. Then, again using the MATLAB command region props, we fitted an ellipse to each remaining region. These ellipses had the same normalized second central moments as the regions to which they were fit. Examples of the ellipses fitted to various pieces of DNA can be seen in Figure 11B. For each realization of DNA relaxation at each frame after electric field was turned off, a collection of fitted ellipses ideally representing the shape of various pieces of DNA at the given time for the given realization were obtained. Figures 21D to 21E and 21I to 21J show examples of the aforementioned island identification and major axes estimation. Figures 21D and 21E show an example for vertex-pinned DNA before relaxation while Figures 21I and 21J show an example for vertex-pinned DNA during relaxation. The ellipses approximation is shown in superposed grey ellipses, and the ellipse major axes are shown using two straight line segments orthogonal to each other. From this collection of fitted ellipses, we recorded the following two values: the mean length of the fitted ellipses' major axis, which we used as the characteristic length of DNA during relaxation; and the standard deviation of major axis length for fitted ellipses whose major axes were longer or shorter than the mean major axis length. The resulting analyses results are shown in Figure 11C and 11D for one and two standard deviations. The black line shows the characteristic length, and the dark gray and light gray shaded regions represent data with one or two standard deviations of the mean major axis length. The only difference between the relaxation analyses of 48.5 and 20 kbp DNA was the threshold chosen to remove selected regions believed to be DNA fragments or image noise.

Contour length of PVP and DNA of multiple MWs

It is noted that unstained $\lambda$-DNA (48.5 kbp) and 20 kbp DNA have reported contour lengths of approximately 16 and 6.6 $\mu$m, respectively. When $\lambda$-DNA and 20 kbp DNA are stained with YOYO-1 dye, their full contour length is extended to be approximately 21 and 8.7 $\mu$m, respectively. PVP of MWs of 10, 58, 360, and 1300 kDa have contour lengths of approximately 25, 145, 900, and 3250 nm, respectively.

Spatial averaging intensity

The symbol $\langle \cdots \rangle$ is used to indicate a spatial averaging over all pixels within a two-dimensional region. For example, to indicate spatial integration of intensity values of a raw image or two-dimensional correlation. For a quantity $q$ for each time frame, the spatial averaged value is then denoted as set forth in Equation (1):

$$\langle q \rangle = \frac{1}{N_x N_y} \sum_{i=1}^{N_x} \sum_{j=1}^{N_y} q_{ij}, \qquad (1)$$

where $N_x$ and $N_y$ indicate the pixel number in the horizontal and vertical directions, respectively.

Estimation of the magnitude of the axial electric field

The conductivity of the buffer as described previously was measured via a commercial conductivity meter. We measured the current during the experiments via a Keithley SourceMeter. We measured the depth of the channel via a profilometer, and the measured depth is approximately 0.9 $\mu$m. The photolithography mask determined the width of the channel, and the width could be measured from the bright field image as shown in as the left most subplot in Figure 9C, which is approximately 20 $\mu$m. The axial electric field strength can be related to the current through Ohmic law as set forth in Equation (2):

$$j = \sigma E \qquad (2)$$

where $j$ is the current density and $\sigma$ is the conductivity of the buffer. The measured current, $I$, was divided by the estimated cross-sectional area, $A$, to obtain the current density, $j$. Then, the electric field strength was estimated by $E = j/\sigma$.

While exemplary embodiments are described above, it is not intended that these embodiments describe all possible forms of the disclosure. Rather, the words used in the specification are words of description rather than limitation, and it is understood that various changes may be made without departing from the spirit and scope of the disclosure. Additionally, the features of various implementing embodiments may be combined to form further embodiments of the disclosure.

**Claims**

1. A method of trapping a polynucleotide strand at a defined surface of a channel comprising:

   providing a device including a channel;
   providing to a defined surface of the channel a polymer capable of modifying an electroosmotic flow in the channel and a sample including a polynucleotide strand;
   physically interacting the polymer with the defined surface of the channel; and
   applying an electric field to the defined surface of the channel to promote a physical interaction between the polynucleotide strand and the polymer to trap the polynucleotide strand at the defined surface of the channel.

2. The method of claim 1, wherein the channel is a microfluidic channel.

3. The method of claim 2, further comprising using flow focusing to direct the polymer to the defined surface of the channel.

4. The method of claim 1, further comprising shaping the channel walls and/or adding features to the channel walls to modify the magnitude of the electric field at the defined surface.

5. The method of claim 1, wherein the defined surface is a dielectric material.

6. The method of claim 5, wherein the dielectric material is an oxide.

7. The method of claim 5, wherein the channel surface outside of the defined surface is a non-oxide material.

8. The method of claim 7, wherein the non-oxide material is a metal.

9. The method of claim 7, wherein the polymer is capable of adsorbing to the oxide material and wherein the polymer is not capable of adsorbing to the non-oxide material.

10. The method of claim 1, wherein the polymer is a neutral water-soluble polymer.

11. The method of claim 1, wherein the polymer is a polyvinylpyrrolidone polymer of greater than 100 kDa.

12. The method of claim 3, wherein the polymer is a neutral water-soluble polymer.

13. The method of claim 3, wherein the polymer is a polyvinylpyrrolidone polymer of greater than 100 kDa.

14. The method of claim 4, wherein the polymer is a neutral water-soluble polymer.

15. The method of claim 4, wherein the polymer is a polyvinylpyrrolidone polymer of greater than 100 kDa.

16. The method of claim 9, wherein the polymer is a neutral water-soluble polymer.

17. The method of claim 9, wherein the polymer is a polyvinylpyrrolidone polymer of greater than 100 kDa.

EP 4 721 861 A1

FIG. 1A

FIG. 1B

110

Provide Sample
to Channel

112

114

Apply an
Electric Field

116

E Field

## FIG. 2A

111

Provide Sample
to Channel

112

114

Apply an
Electric Field

116

E Field

Modify
Electric
Field

117

## FIG. 2B

**FIG. 3A**

**FIG. 3B**

*126*

*128a*

Using Flow Focusing to Coat Only Certain Area of Channel
Substrate With PVP Where Vertex-pinning Can Happen

Flow Without PVP

*128b*

Flow With PVP

Flow Without PVP

*128c*

**FIG. 4**

*130*

*132a*
Low E Field Region

*134*
High E Field Region
For DNA Vertex Trapping

*132b*
Low E Field Region

E Field

**FIG. 5**

**FIG. 6**

FIG. 7A

*146*

PROVIDING TO THE CHANNEL A POLYMER CAPABLE OF MODIFYING AN ELECTROOSMOTIC FLOW IN THE CHANNEL *147*

PROVIDING A SAMPLE INCLUDING THE LABELED POLYNUCLEOTIDE STRAND TO THE CHANNEL *148*

APPLYING AN ELECTRIC FIELD TO THE LABELED POLYNUCLEOTIDE STRANDS IN THE CHANNEL *150*

IMAGING THE LABELED POLYNUCLEOTIDE STRANDS *152*

# FIG. 7B

**FIG. 8**

FIG. 9A

FIG. 9B

FIG. 9C

EP 4 721 861 A1

Moving Median Image

Threshold Followed by Alpha Shape
Bounding to Create the Mask

Mask

Mask Applied to Image, then Integration

Masked Image

20 µm

## FIG. 10A

## FIG. 10B

FIG. 11B

FIG. 11A

**FIG. 11D**

20 kbp DNA
2% w/w 1300 kDa PVP

Time After Electric Field is Deactivated, $t$ [s]

Characteristic Length of the Selected DNA Molecule, $L$ [μm]

2 St. Dev.
St. Dev.
Mean

**FIG. 11C**

48.5 kbp λ-DNA
2% w/w 1300 kDa PVP

Time After Electric Field is Deactivated, $t$ [s]

Characteristic Length of the Selected DNA Molecule, $L$ [μm]

2 St. Dev.
St. Dev.
Mean

time

E. Field of 190 V/cm
Applied at t = 2.4s to
Initiate Trapping.

High E. Field Causes
Further Trapping.

E. Field Removed
at t = 33.8s and Some
Trapped DNA Released
Quickly While Some
Trapped DNA Relaxes
Slowly Toward Vertices.

DNA Molecules Relax and
Become Disentangled.

E. Field of 11 V/cm Applied
at t = 88.6 and DNA
Molecules Are Free to
Electromigrate .

System Now Ready for
New Realization of
DNA Trapping.

t = 0    E = 0
t = 3.1s    ← E = 190 V/cm
t = 33.4s    ← E = 190 V/cm
t = 33.9s
t = 34.2s
t = 34.5s    E = 0
t = 40.6s    E = 0
t = 88.6s    ← E = 11 V/cm
t = 89.5s    ← E = 11 V/cm
t = 90.2s    ← E = 11 V/cm
20 µm

## FIG. 12

Realization 1

FIG. 13A

Realization 2

FIG. 13B

Realization 3

FIG. 13C

FIG. 13D

FIG. 13E

FIG. 13F

FIG. 13G

PDMS
Reservoir

Borosilicate
Glass

Silicon
Substrate
Chip

**FIG. 14A**

Assembled
Device

**FIG. 14B**

**FIG. 14C**

FIG. 14E

10 μm

FIG. 14F

50 μm

FIG. 14D

5 μm

14E

14F

**FIG. 15A**

**FIG. 15B**

Schematic of the Commercial Glass Chip

58.5 mm

**FIG. 16A**

|E| = 256 V/cm        t = 0

**FIG. 16B**

←  |E| = 256 V/cm        t = 0

**FIG. 16C**

Global Temporal Median

20 μm

**FIG. 16D**

38

**FIG. 17**

FIG. 18A

FIG. 18B

FIG. 18D

FIG. 18C

**FIG. 19**

**FIG. 20A**

**FIG. 20B**

EP 4 721 861 A1

**FIG. 20C**

**FIG. 20D**

*t* = 0s     1.0s     1.5s     2.0s     5.0s

15 µm

# FIG. 21

Vertex-pinned 48.5 kbp λ-DNA, 2% w/w 1300 kDa PVP

FIG. 22A

FIG. 22B

FIG. 22C

FIG. 22D

FIG. 22E

Relaxing 48.5 kbp DNA, 2% w/w 1300 kDa PVP

FIG. 22F

FIG. 22G

FIG. 22H

FIG. 22I

FIG. 22J

Vertex-pinned 48.5 kbp λ-DNA, 2% w/w 1300 kDa PVP

FIG. 23A

FIG. 23B

FIG. 23C

FIG. 23D

FIG. 23E

FIG. 23F

FIG. 23G

FIG. 23H

FIG. 23I

FIG. 23J

Relaxing 20 kbp DNA, 2% w/w 1300 kDa PVP

FIG. 24A

FIG. 24B

FIG. 24C

$t_1 = 21.0s$　　　$t_2 = 26.0s$　　　$t_1 = 28.0s$

20 µm

**FIG. 25A**

FIG. 25B

FIG. 25C

Raw Video
21.40s
E = 68.5 V/cm ←
15 µm

Moving Median Video
21.40s
E = 68.5 V/cm ←
15 µm

Comparison Between Raw Video and Processed Moving Median Video. The Latter Removes Moving DNA Molecules and Amplifies Vertex-pinned Ones.

Raw Video
23.35s
E = 68.5 V/cm ←
15 µm

Moving Median Video
23.35s
E = 68.5 V/cm ←
15 µm

Comparison Between Raw Video and Processed Moving Median Video. The Latter Removes Moving DNA Molecules and Amplifies Vertex-pinned Ones.

**FIG. 26**

Europäisches
Patentamt
European
Patent Office
Office européen
des brevets

# EUROPEAN SEARCH REPORT

**Application Number**

EP 25 20 6039

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RYAN J MONTES ET AL: "Trapping DNA with a high throughput microfluidic device", ELECTROPHORESIS, VERLAG CHEMIE, HOBOKEN, USA, vol. 40, no. 3, 10 October 2018 (2018-10-10), pages 437-446, XP071504822, ISSN: 0173-0835, DOI: 10.1002/ELPS.201800287 | 1-6, 10-15,17 | INV. B01L3/00 C12Q1/6806 |
| Y | * pages 438, 439, paragraphs 2, 2.1, 2.2, 2.3; figures 1,2 * ----- | 7-9,16 | |
| X | WO 2006/052882 A1 (HARVARD COLLEGE [US]; PARK STELLA [US] ET AL.) 18 May 2006 (2006-05-18) | 1-6, 10-15,17 | |
| Y | * page 28, lines 13-20; claim 1; figure 1 * <br> * page 24, lines 19-30; claim 3 * <br> * page 21, lines 15-17 * ----- | 7-9,16 | |
| Y | US 2023/138703 A1 (ZHOU JING [US] ET AL) 4 May 2023 (2023-05-04) * paragraphs [0058], [0071]; figures 1A, 1C, 5A-5E * ----- | 7-9,16 | **TECHNICAL FIELDS SEARCHED (IPC)** <br><br> B01L <br> C12Q |

The present search report has been drawn up for all claims

| Place of search | Date of completion of the search | Examiner |
|---|---|---|
| The Hague | 6 February 2026 | Campbell, Paul |

EPO FORM 1503 03.82 (P4C01)

**EP 4 721 861 A1**

## ANNEX TO THE EUROPEAN SEARCH REPORT
## ON EUROPEAN PATENT APPLICATION NO.

EP 25 20 6039

06-02-2026

| Patent document cited in search report | Publication date | Patent family member(s) | | Publication date |
|---|---|---|---|---|
| WO 2006052882 A1 | 18-05-2006 | NONE | | |
| US 2023138703 A1 | 04-05-2023 | CN | 118139692 A | 04-06-2024 |
| | | EP | 4392176 A1 | 03-07-2024 |
| | | US | 2023138703 A1 | 04-05-2023 |
| | | WO | 2023028582 A1 | 02-03-2023 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63702095